(19) Europäisches Patentamt
European Patent Office
Office européen des brevets

(11) **EP 3 234 598 B1**

(12) **EUROPEAN PATENT SPECIFICATION**

(45) Date of publication and mention
of the grant of the patent:
**06.11.2019 Bulletin 2019/45**

(51) Int Cl.:
*G01N 33/50* (2006.01)    *G01N 33/68* (2006.01)

(21) Application number: **15817155.3**

(86) International application number:
**PCT/EP2015/079693**

(22) Date of filing: **15.12.2015**

(87) International publication number:
**WO 2016/096788 (23.06.2016 Gazette 2016/25)**

(54) **ASSAY AND METHOD FOR DETERMINING CDC ELICITING ANTIBODIES**

ASSAY UND VERFAHREN ZUR BESTIMMUNG CDC-ENTLOCKENDER ANTIKÖRPER

DOSAGE ET PROCÉDÉ DE DÉTERMINATION DE CDC PRODUISANT DES ANTICORPS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2014 EP 14198749**

(43) Date of publication of application:
**25.10.2017 Bulletin 2017/43**

(73) Proprietor: **F. Hoffmann-La Roche AG
4070 Basel (CH)**

(72) Inventors:
• **OFFNER, Sonja**
  **82377 Penzberg (DE)**
• **ZICK, Karlheinz**
  **81739 Muenchen (DE)**

(74) Representative: **Burger, Alexander et al
Roche Diagnostics GmbH
Patentabteilung
Nonnenwald 2
82377 Penzberg (DE)**

(56) References cited:
• **E. KONISHI ET AL: "Utilization of
Complement-Dependent Cytotoxicity To
Measure Low Levels of Antibodies: Application
to Nonstructural Protein 1 in a Model of Japanese
Encephalitis Virus", CLINICAL AND VACCINE
IMMUNOLOGY, vol. 15, no. 1, 1 January 2008
(2008-01-01), pages 88-94, XP055194781, ISSN:
1556-6811, DOI: 10.1128/CVI.00347-07**

• **KLITGAARD JOSEPHINE L ET AL: "Combination
of two anti-CD5 monoclonal antibodies
synergistically induces complement-dependent
cytotoxicity of chronic lymphocytic leukaemia
cells", BRITISH JOURNAL OF HAEMATOLOGY,
vol. 163, no. 2, October 2013 (2013-10), pages
182-193, XP055195519,**

• **HELLSTRÖM I ET AL: "Monoclonal antibodies to
two determinants of melanoma-antigen p97 act
synergistically in complement-dependent
cytotoxicity.", JOURNAL OF IMMUNOLOGY
(BALTIMORE, MD. : 1950) JUL 1981, vol. 127, no.
1, July 1981 (1981-07), pages 157-160,
XP055195523, ISSN: 0022-1767**

• **MADDIPATLA S ET AL: "Augmented antitumor
activity against B-cell lymphoma by a
combination of monoclonal antibodies targeting
TRAIL-R1 and CD20", CLINICAL CANCER
RESEARCH 20070801 US, vol. 13, no. 15, 1 August
2007 (2007-08-01), pages 4556-4564,
XP055195526, ISSN: 1078-0432**

• **HUANG JIAN ET AL: "Protection of xenogENEIC
cells from human complement-mediated lysis by
the expression of human DAF, CD59 and MCP",
FEMS IMMUNOLOGY AND MEDICAL
MICROBIOLOGY, ELSEVIER SCIENCE B.V.,
AMSTERDAM, NL, vol. 31, no. 3, 1 October 2001
(2001-10-01), pages 203-209, XP002249602, ISSN:
0928-8244, DOI: 10.1016/S0928-8244(01)00261-9**

• **QU ZHENGXING ET AL: "Recombinant bispecific
monoclonal antibody (bsMAb) against CD20 and
CD22 active in vitro and in vivo against B-cell
lymphomas", BLOOD, AMERICAN SOCIETY OF
HEMATOLOGY, US, vol. 108, no. 11, part 1, 1
November 2006 (2006-11-01), pages 713A-714A,
XP008176628, ISSN: 0006-4971**

SEQUENCE LISTING

<110> F. Hoffmann-La Roche AG

<120> ASSAY AND METHOD FOR DETERMINING CDC ELICITING ANTIBODIES

<130> P32471-WO

<150> EP14198749.5
<151> 2014-12-18

<160> 19

<170> PatentIn version 3.5

<210> 1
<211> 214
<212> PRT
<213> Artificial Sequence

<220>
<223> trastuzumab LC

<400> 1

```
Asp Ile Gln Met Thr Gln Ser Pro Ser Ser Leu Ser Ala Ser Val Gly
1               5               10              15

Asp Arg Val Thr Ile Thr Cys Arg Ala Ser Gln Asp Val Asn Thr Ala
            20              25              30

Val Ala Trp Tyr Gln Gln Lys Pro Gly Lys Ala Pro Lys Leu Leu Ile
        35              40              45

Tyr Ser Ala Ser Phe Leu Tyr Ser Gly Val Pro Ser Arg Phe Ser Gly
    50              55              60

Ser Arg Ser Gly Thr Asp Phe Thr Leu Thr Ile Ser Ser Leu Gln Pro
65              70              75              80

Glu Asp Phe Ala Thr Tyr Tyr Cys Gln Gln His Tyr Thr Thr Pro Pro
            85              90              95

Thr Phe Gly Gln Gly Thr Lys Val Glu Ile Lys Arg Thr Val Ala Ala
        100             105             110

Pro Ser Val Phe Ile Phe Pro Pro Ser Asp Glu Gln Leu Lys Ser Gly
        115             120             125

Thr Ala Ser Val Val Cys Leu Leu Asn Asn Phe Tyr Pro Arg Glu Ala
    130             135             140

Lys Val Gln Trp Lys Val Asp Asn Ala Leu Gln Ser Gly Asn Ser Gln
145             150             155             160

Glu Ser Val Thr Glu Gln Asp Ser Lys Asp Ser Thr Tyr Ser Leu Ser
            165             170             175

Ser Thr Leu Thr Leu Ser Lys Ala Asp Tyr Glu Lys His Lys Val Tyr
        180             185             190

Ala Cys Glu Val Thr His Gln Gly Leu Ser Ser Pro Val Thr Lys Ser
        195             200             205

Phe Asn Arg Gly Glu Cys
        210
```

<210> 2
<211> 450
<212> PRT
<213> Artificial Sequence

<220>

<223> trastuzumab HC

<400> 2

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
            115                 120                 125

Phe Pro Leu Ala Pro Ser Ser Lys Ser Thr Ser Gly Gly Thr Ala Ala
            130                 135                 140
```

```
Leu Gly Cys Leu Val Lys Asp Tyr Phe Pro Glu Pro Val Thr Val Ser
145                 150             155                 160

Trp Asn Ser Gly Ala Leu Thr Ser Gly Val His Thr Phe Pro Ala Val
                165             170             175

Leu Gln Ser Ser Gly Leu Tyr Ser Leu Ser Ser Val Val Thr Val Pro
            180             185             190

Ser Ser Ser Leu Gly Thr Gln Thr Tyr Ile Cys Asn Val Asn His Lys
        195             200             205

Pro Ser Asn Thr Lys Val Asp Lys Lys Val Glu Pro Lys Ser Cys Asp
    210             215             220

Lys Thr His Thr Cys Pro Pro Cys Pro Ala Pro Glu Leu Leu Gly Gly
225             230             235             240

Pro Ser Val Phe Leu Phe Pro Pro Lys Pro Lys Asp Thr Leu Met Ile
            245             250             255

Ser Arg Thr Pro Glu Val Thr Cys Val Val Val Asp Val Ser His Glu
        260             265             270

Asp Pro Glu Val Lys Phe Asn Trp Tyr Val Asp Gly Val Glu Val His
    275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315             320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Glu Glu Met Thr Lys Asn Gln Val Ser Leu
        355             360             365

Thr Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395             400
```

27

```
Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
                420                 425                 430

Glu Ala Leu His Asn His Tyr Thr Gln Lys Ser Leu Ser Leu Ser Pro
                435                 440                 445

Gly Lys
450
```

<210> 3
<211> 214
<212> PRT
<213> Artificial Sequence

<220>
<223> pertuzumab LC

<400> 3

```
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Ile Gly
                20                  25                  30

Val Ala Trp Tyr Gln Gln Lys Pro Gly Lys Ala Pro Lys Leu Leu Ile
                35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile Lys Arg Thr Val Ala Ala
                100                 105                 110

Pro Ser Val Phe Ile Phe Pro Pro Ser Asp Glu Gln Leu Lys Ser Gly
                115                 120                 125

Thr Ala Ser Val Val Cys Leu Leu Asn Asn Phe Tyr Pro Arg Glu Ala
                130                 135                 140
```

```
Lys Val Gln Trp Lys Val Asp Asn Ala Leu Gln Ser Gly Asn Ser Gln
145             150             155             160

Glu Ser Val Thr Glu Gln Asp Ser Lys Asp Ser Thr Tyr Ser Leu Ser
                165             170             175

Ser Thr Leu Thr Leu Ser Lys Ala Asp Tyr Glu Lys His Lys Val Tyr
                180             185             190

Ala Cys Glu Val Thr His Gln Gly Leu Ser Ser Pro Val Thr Lys Ser
        195             200             205

Phe Asn Arg Gly Glu Cys
        210
```

<210> 4
<211> 449
<212> PRT
<213> Artificial Sequence

<220>
<223> pertuzumab HC

<400> 4

```
Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Thr Asp Tyr
                20              25              30

Thr Met Asp Trp Val Arg Gln Ala Pro Gly Lys Gly Leu Glu Trp Val
            35              40              45

Ala Asp Val Asn Pro Asn Ser Gly Gly Ser Ile Tyr Asn Gln Arg Phe
        50              55              60

Lys Gly Arg Phe Thr Leu Ser Val Asp Arg Ser Lys Asn Thr Leu Tyr
65              70              75              80

Leu Gln Met Asn Ser Leu Arg Ala Glu Asp Thr Ala Val Tyr Tyr Cys
                85              90              95

Ala Arg Asn Leu Gly Pro Ser Phe Tyr Phe Asp Tyr Trp Gly Gln Gly
                100             105             110

Thr Leu Val Thr Val Ser Ser Ala Ser Thr Lys Gly Pro Ser Val Phe
            115             120             125
```

29

```
Pro Leu Ala Pro Ser Ser Lys Ser Thr Ser Gly Gly Thr Ala Ala Leu
    130             135             140

Gly Cys Leu Val Lys Asp Tyr Phe Pro Glu Pro Val Thr Val Ser Trp
    145             150             155             160

Asn Ser Gly Ala Leu Thr Ser Gly Val His Thr Phe Pro Ala Val Leu
                165             170             175

Gln Ser Ser Gly Leu Tyr Ser Leu Ser Ser Val Val Thr Val Pro Ser
            180             185             190

Ser Ser Leu Gly Thr Gln Thr Tyr Ile Cys Asn Val Asn His Lys Pro
        195             200             205

Ser Asn Thr Lys Val Asp Lys Lys Val Glu Pro Lys Ser Cys Asp Lys
    210             215             220

Thr His Thr Cys Pro Pro Cys Pro Ala Pro Glu Leu Leu Gly Gly Pro
225             230             235             240

Ser Val Phe Leu Phe Pro Pro Lys Pro Lys Asp Thr Leu Met Ile Ser
                245             250             255

Arg Thr Pro Glu Val Thr Cys Val Val Val Asp Val Ser His Glu Asp
            260             265             270

Pro Glu Val Lys Phe Asn Trp Tyr Val Asp Gly Val Glu Val His Asn
            275             280             285

Ala Lys Thr Lys Pro Arg Glu Glu Gln Tyr Asn Ser Thr Tyr Arg Val
    290             295             300

Val Ser Val Leu Thr Val Leu His Gln Asp Trp Leu Asn Gly Lys Glu
305             310             315             320

Tyr Lys Cys Lys Val Ser Asn Lys Ala Leu Pro Ala Pro Ile Glu Lys
                325             330             335

Thr Ile Ser Lys Ala Lys Gly Gln Pro Arg Glu Pro Gln Val Tyr Thr
            340             345             350

Leu Pro Pro Ser Arg Asp Glu Leu Thr Lys Asn Gln Val Ser Leu Thr
            355             360             365

Cys Leu Val Lys Gly Phe Tyr Pro Ser Asp Ile Ala Val Glu Trp Glu
    370             375             380
```

30

```
Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
                420                 425                 430

Ala Leu His Asn His Tyr Thr Gln Lys Ser Leu Ser Leu Ser Pro Gly
                435                 440                 445

Lys
```

<210> 5
<211> 214
<212> PRT
<213> Artificial Sequence

<220>
<223> common light chain

<400> 5

```
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Thr Ala
                20                  25                  30

Val Ala Trp Tyr Gln Gln Lys Pro Gly Lys Ala Pro Lys Leu Leu Ile
                35                  40                  45

Tyr Ser Ala Ser Phe Arg Tyr Thr Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
                100                 105                 110

Pro Ser Val Phe Ile Phe Pro Pro Ser Asp Glu Gln Leu Lys Ser Gly
                115                 120                 125
```

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
        210
```

<210> 6
<211> 450
<212> PRT
<213> Artificial Sequence

<220>
<223> trastuzumab HC knob

<400> 6

Glu Val Gln Leu Val Glu Ser Gly Gly Gly Leu Val Gln Pro Gly Gly
1               5               10              15

Ser Leu Arg Leu Ser Cys Ala Ala Ser Gly Phe Asn Ile Lys Asp Thr
         20                  25                  30

Tyr Ile His Trp Val Arg Gln Ala Pro Gly Lys Gly Leu Glu Trp Val
         35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
             85                  90                  95

Ser Arg Trp Gly Gly Asp Gly Phe Tyr Ala Met Asp Tyr Trp Gly Gln
         100                 105                 110

Gly Thr Leu Val Thr Val Ser Ser Ala Ser Thr Lys Gly Pro Ser Val

|  |  |  | 115 |  |  |  |  | 120 |  |  |  |  | 125 |  |  |
|--|--|--|--|--|--|--|--|--|--|--|--|--|--|--|--|

Phe Pro Leu Ala Pro Ser Ser Lys Ser Thr Ser Gly Gly Thr Ala Ala
    130             135             140

Leu Gly Cys Leu Val Lys Asp Tyr Phe Pro Glu Pro Val Thr Val Ser
145             150             155             160

Trp Asn Ser Gly Ala Leu Thr Ser Gly Val His Thr Phe Pro Ala Val
            165             170             175

Leu Gln Ser Ser Gly Leu Tyr Ser Leu Ser Ser Val Val Thr Val Pro
        180             185             190

Ser Ser Ser Leu Gly Thr Gln Thr Tyr Ile Cys Asn Val Asn His Lys
    195             200             205

Pro Ser Asn Thr Lys Val Asp Lys Lys Val Glu Pro Lys Ser Cys Asp
    210             215             220

Lys Thr His Thr Cys Pro Pro Cys Pro Ala Pro Glu Leu Leu Gly Gly
225             230             235             240

Pro Ser Val Phe Leu Phe Pro Pro Lys Pro Lys Asp Thr Leu Met Ile
            245             250             255

Ser Arg Thr Pro Glu Val Thr Cys Val Val Val Asp Val Ser His Glu
        260             265             270

Asp Pro Glu Val Lys Phe Asn Trp Tyr Val Asp Gly Val Glu Val His
        275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315             320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
        340             345             350

Thr Leu Pro Pro Cys Arg Asp Glu Leu Thr Lys Asn Gln Val Ser Leu
    355             360             365

```
Trp Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395             400

Leu Asp Ser Asp Gly Ser Phe Phe Leu Tyr Ser Lys Leu Thr Val Asp
                405             410             415

Lys Ser Arg Trp Gln Gln Gly Asn Val Phe Ser Cys Ser Val Met His
            420             425             430

Glu Ala Leu His Asn His Tyr Thr Gln Lys Ser Leu Ser Leu Ser Pro
        435             440             445

Gly Lys
    450
```

<210> 7
<211> 449
<212> PRT
<213> Artificial Sequence

<220>
<223> pertuzumab HC hole

<400> 7

Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Asn Asp Tyr
            20              25              30

Thr Met Asp Trp Val Arg Gln Ala Pro Gly Lys Gly Leu Glu Trp Val
        35              40              45

Ala Asp Val Asn Pro Asn Ser Gly Gly Ser Ile Val Asn Arg Arg Phe
    50              55              60

Lys Gly Arg Phe Thr Leu Ser Val Asp Arg Ser Lys Asn Thr Leu Tyr
65              70              75              80

Leu Gln Met Asn Ser Leu Arg Ala Glu Asp Thr Ala Val Tyr Tyr Cys
            85              90              95

Ala Arg Asn Leu Gly Pro Phe Phe Tyr Phe Asp Tyr Trp Gly Gln Gly
        100             105             110

Thr Leu Val Thr Val Ser Ser Ala Ser Thr Lys Gly Pro Ser Val Phe

| | | 115 | | | | | 120 | | | | | | 125 | | | |

Pro Leu Ala Pro Ser Ser Lys Ser Thr Ser Gly Gly Thr Ala Ala Leu
    130        135        140

Gly Cys Leu Val Lys Asp Tyr Phe Pro Glu Pro Val Thr Val Ser Trp
    145        150        155        160

Asn Ser Gly Ala Leu Thr Ser Gly Val His Thr Phe Pro Ala Val Leu
        165        170        175

Gln Ser Ser Gly Leu Tyr Ser Leu Ser Ser Val Val Thr Val Pro Ser
        180        185        190

Ser Ser Leu Gly Thr Gln Thr Tyr Ile Cys Asn Val Asn His Lys Pro
        195        200        205

Ser Asn Thr Lys Val Asp Lys Lys Val Glu Pro Lys Ser Cys Asp Lys
    210        215        220

Thr His Thr Cys Pro Pro Cys Pro Ala Pro Glu Leu Leu Gly Gly Pro
225        230        235        240

Ser Val Phe Leu Phe Pro Pro Lys Pro Lys Asp Thr Leu Met Ile Ser
        245        250        255

Arg Thr Pro Glu Val Thr Cys Val Val Val Asp Val Ser His Glu Asp
        260        265        270

Pro Glu Val Lys Phe Asn Trp Tyr Val Asp Gly Val Glu Val His Asn
        275        280        285

Ala Lys Thr Lys Pro Arg Glu Glu Gln Tyr Asn Ser Thr Tyr Arg Val
        290        295        300

Val Ser Val Leu Thr Val Leu His Gln Asp Trp Leu Asn Gly Lys Glu
305        310        315        320

Tyr Lys Cys Lys Val Ser Asn Lys Ala Leu Pro Ala Pro Ile Glu Lys
        325        330        335

Thr Ile Ser Lys Ala Lys Gly Gln Pro Arg Glu Pro Gln Val Cys Thr
        340        345        350

Leu Pro Pro Ser Arg Asp Glu Leu Thr Lys Asn Gln Val Ser Leu Ser
        355        360        365

```
        Cys Ala Val Lys Gly Phe Tyr Pro Ser Asp Ile Ala Val Glu Trp Glu
            370             375             380

        Ser Asn Gly Gln Pro Glu Asn Asn Tyr Lys Thr Thr Pro Pro Val Leu
        385             390             395             400

        Asp Ser Asp Gly Ser Phe Phe Leu Val Ser Lys Leu Thr Val Asp Lys
                        405             410             415

        Ser Arg Trp Gln Gln Gly Asn Val Phe Ser Cys Ser Val Met His Glu
                    420             425             430

        Ala Leu His Asn His Tyr Thr Gln Lys Ser Leu Ser Leu Ser Pro Gly
                    435             440             445

        Lys
```

<210> 8
<211> 450
<212> PRT
<213> Artificial Sequence

<220>
<223> CrossMab HC1

<400> 8

```
Gln Val Gln Leu Val Gln Ser Gly Ala Glu Val Lys Lys Pro Gly Ala
1               5               10              15

Ser Val Lys Val Ser Cys Lys Ala Ser Gly Phe Asn Ile Lys Asp Thr
            20              25              30

Tyr Ile His Trp Val Arg Gln Ala Pro Gly Gln Gly Leu Glu Trp Met
        35              40              45

Gly Arg Ile Tyr Pro Thr Asn Gly Tyr Thr Arg Tyr Ala Gln Lys Phe
    50              55              60

Gln Gly Arg Val Thr Met Thr Arg Asp Thr Ser Ile Ser Thr Ala Tyr
65              70              75              80

Met Glu Leu Ser Arg Leu Arg Ser Asp Asp Thr Ala Val Tyr Tyr Cys
            85              90              95

Ser Arg Trp Gly Gly Glu Gly Phe Tyr Ala Met Asp Tyr Trp Gly Gln
        100             105             110

Gly Thr Met Val Thr Val Ser Ser Ala Ser Thr Lys Gly Pro Ser Val
```

```
                    115                    120                    125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
                180                 185                 190

Ser Ser Ser Leu Gly Thr Gln Thr Tyr Ile Cys Asn Val Asn His Lys
            195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
            275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Cys
            340                 345                 350

Thr Leu Pro Pro Ser Arg Asp Glu Leu Thr Lys Asn Gln Val Ser Leu
    355                 360                 365
```

```
Ser Cys Ala Val Lys Gly Phe Tyr Pro Ser Asp Ile Ala Val Glu Trp
    370                 375             380

Glu Ser Asn Gly Gln Pro Glu Asn Asn Tyr Lys Thr Thr Pro Pro Val
385                 390             395                     400

Leu Asp Ser Asp Gly Ser Phe Phe Leu Val Ser Lys Leu Thr Val Asp
                405             410                 415

Lys Ser Arg Trp Gln Gln Gly Asn Val Phe Ser Cys Ser Val Met His
            420             425             430

Glu Ala Leu His Asn His Tyr Thr Gln Lys Ser Leu Ser Leu Ser Pro
        435             440             445

Gly Lys
    450
```

<210> 9
<211> 453
<212> PRT
<213> Artificial Sequence

<220>
<223> CrossMab HC2

<400> 9

Glu Val Gln Leu Val Glu Ser Gly Gly Gly Leu Val Gln Pro Gly Gly
1             5                 10                 15

Ser Leu Arg Leu Ser Cys Ala Ala Ser Gly Phe Thr Phe Thr Asp Tyr
            20                  25                 30

Thr Met Asp Trp Val Arg Gln Ala Pro Gly Lys Gly Leu Glu Trp Val
            35                  40                 45

Ala Asp Val Asn Pro Asn Ser Gly Gly Ser Ile Tyr Asn Gln Arg Phe
        50                  55                 60

Lys Gly Arg Phe Thr Leu Ser Val Asp Arg Ser Lys Asn Thr Leu Tyr
65                  70                  75                 80

Leu Gln Met Asn Ser Leu Arg Ala Glu Asp Thr Ala Val Tyr Tyr Cys
                85                  90                 95

Ala Arg Asn Leu Gly Pro Ser Phe Tyr Phe Asp Tyr Trp Gly Gln Gly
            100                 105                110

Thr Leu Val Thr Val Ser Ser Ala Ser Val Ala Ala Pro Ser Val Phe

**42**

                        115                     120                     125

Ile Phe Pro Pro Ser Asp Glu Gln Leu Lys Ser Gly Thr Ala Ser Val
    130             135             140

Val Cys Leu Leu Asn Asn Phe Tyr Pro Arg Glu Ala Lys Val Gln Trp
145             150             155             160

Lys Val Asp Asn Ala Leu Gln Ser Gly Asn Ser Gln Glu Ser Val Thr
            165             170             175

Glu Gln Asp Ser Lys Asp Ser Thr Tyr Ser Leu Ser Ser Thr Leu Thr
            180             185             190

Leu Ser Lys Ala Asp Tyr Glu Lys His Lys Val Tyr Ala Cys Glu Val
        195             200             205

Thr His Gln Gly Leu Ser Ser Pro Val Thr Lys Ser Phe Asn Arg Gly
    210             215             220

Glu Cys Asp Lys Thr His Thr Cys Pro Pro Cys Pro Ala Pro Glu Leu
225             230             235             240

Leu Gly Gly Pro Ser Val Phe Leu Phe Pro Pro Lys Pro Lys Asp Thr
            245             250             255

Leu Met Ile Ser Arg Thr Pro Glu Val Thr Cys Val Val Val Asp Val
        260             265             270

Ser His Glu Asp Pro Glu Val Lys Phe Asn Trp Tyr Val Asp Gly Val
    275             280             285

Glu Val His Asn Ala Lys Thr Lys Pro Arg Glu Glu Gln Tyr Asn Ser
    290             295             300

Thr Tyr Arg Val Val Ser Val Leu Thr Val Leu His Gln Asp Trp Leu
305             310             315             320

Asn Gly Lys Glu Tyr Lys Cys Lys Val Ser Asn Lys Ala Leu Pro Ala
            325             330             335

Pro Ile Glu Lys Thr Ile Ser Lys Ala Lys Gly Gln Pro Arg Glu Pro
        340             345             350

Gln Val Tyr Thr Leu Pro Pro Cys Arg Asp Glu Leu Thr Lys Asn Gln
    355             360             365

```
Val Ser Leu Trp Cys Leu Val Lys Gly Phe Tyr Pro Ser Asp Ile Ala
    370             375             380

Val Glu Trp Glu Ser Asn Gly Gln Pro Glu Asn Asn Tyr Lys Thr Thr
385             390             395                 400

Pro Pro Val Leu Asp Ser Asp Gly Ser Phe Phe Leu Tyr Ser Lys Leu
                405             410                 415

Thr Val Asp Lys Ser Arg Trp Gln Gln Gly Asn Val Phe Ser Cys Ser
            420             425                 430

Val Met His Glu Ala Leu His Asn His Tyr Thr Gln Lys Ser Leu Ser
        435             440             445

Leu Ser Pro Gly Lys
        450
```

<210> 10
<211> 213
<212> PRT
<213> Artificial Sequence

<220>
<223> CrossMab LC1

<400> 10

```
Asp Ile Gln Leu Thr Gln Pro Pro Ser Val Ser Val Ala Pro Gly Gln
1               5               10              15

Thr Ala Arg Ile Thr Cys Gly Ala Ser Gln Asp Val Ser Thr Ala Val
            20              25              30

Ala Trp Tyr Gln Gln Lys Pro Gly Gln Ala Pro Val Leu Val Val Tyr
        35              40              45

Ser Ala Ser Phe Leu Tyr Ser Gly Ile Pro Ser Arg Phe Ser Gly Ser
    50              55              60

Arg Ser Gly Thr Asp Phe Thr Leu Thr Ile Ser Arg Val Glu Ala Gly
65              70              75              80

Asp Glu Ala Asp Tyr Tyr Cys Gln Gln His Tyr Thr Thr Pro Pro Thr
            85              90              95

Phe Gly Thr Gly Thr Lys Val Thr Val Leu Arg Thr Val Ala Ala Pro
            100             105             110

Ser Val Phe Ile Phe Pro Pro Ser Asp Glu Gln Leu Lys Ser Gly Thr

        115             120             125

Ala Ser Val Val Cys Leu Leu Asn Asn Phe Tyr Pro Arg Glu Ala Lys
    130             135             140

Val Gln Trp Lys Val Asp Asn Ala Leu Gln Ser Gly Asn Ser Gln Glu
145             150             155             160

Ser Val Thr Glu Gln Asp Ser Lys Asp Ser Thr Tyr Ser Leu Ser Ser
            165             170             175

Thr Leu Thr Leu Ser Lys Ala Asp Tyr Glu Lys His Lys Val Tyr Ala
            180             185             190

Cys Glu Val Thr His Gln Gly Leu Ser Ser Pro Val Thr Lys Ser Phe
            195             200             205

Asn Arg Gly Glu Cys
            210
```

<210> 11
<211> 212
<212> PRT
<213> Artificial Sequence

<220>
<223> CrossMab LC2

<400> 11

```
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Ile Gly
            20                  25                  30

Val Ala Trp Tyr Gln Gln Lys Pro Gly Lys Ala Pro Lys Leu Leu Ile
            35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
            115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
            195                 200                 205

Pro Lys Ser Cys
            210
```

<210> 12

<211> 19
<212> RNA
<213> Artificial Sequence

<220>
<223> siRNA CD46-1

<400> 12
gagagagcac gauuuauug        19

<210> 13
<211> 19
<212> RNA
<213> Artificial Sequence

<220>
<223> siRNA CD46-2

<400> 13
caauaaaucg ugcucucuc        19

<210> 14
<211> 19
<212> RNA
<213> Artificial Sequence

<220>
<223> siRNA CD55-1

<400> 14
gaagaguucu gcaaucgua        19

<210> 15
<211> 19
<212> RNA
<213> Artificial Sequence

<220>
<223> siRNA CD55-2

<400> 15
uacgauugca gaacucuuc        19

<210> 16
<211> 19
<212> RNA
<213> Artificial Sequence

<220>
<223> siRNA CD59-1

<400> 16
gcaagaagga ccuguguaa        19

<210> 17
<211> 19
<212> RNA
<213> Artificial Sequence

<220>
<223> siRNA CD59-2

<400> 17
uuacacaggu ccuucuugc        19

<210> 18
<211> 19
<212> RNA
<213> Artificial Sequence

<220>
<223> scrambled siRNA-1

<400> 18
ugcagguuua uaguccaca        19

<210> 19
<211> 19
<212> RNA
<213> Artificial Sequence

<220>
<223> scrambled siRNA-2

<400> 19
uguggacuau aaaccugca        19

## Description

## FIELD OF THE INVENTION

The current invention is in the field of assays and methods for the detection/selection of effector function eliciting antibodies and antibody combinations.

## BACKGROUND

Immunoglobulins contain two binding sites for certain Fc receptors, such as FcRn, as well as for Clq, one in each heavy chain Fc-region.

For complement activation more than a single immunoglobulin molecule is required as the affinity of monomeric IgG for C1q is quite weak (affinity about $10^{-4}$ M) (see e.g. Sledge et al., J. Biol. Chem. 248 (1973) 2818-2813, Hughes-Jones et al., Mol. Immunol. 16 (1979) 697-701). The binding of the multivalent C1q may be increased by antigen-based association of the immunoglobulin molecules and, thus, complement activation (affinity about $10^{-8}$ M) (see e.g. Burton et al., Mol. Immunol. 22 (1990) 161-206).

The three dimensional structure of C1q is like a bunch of tulips comprising six globular heads, which comprise the antibody binding regions (see e.g. Perkins et al., Biochem. J. 228 (1985) 13-26, Poon et al., J. Mol. Biol. 168 (1983) 563-577, Reid et al., Biochem. Soc. Trans. 11 (1983) 1-12, and Weiss et al., J. Mol. Biol. 189 (1986) 573-581).

In US 5,851,528 are reported methods of inhibiting complement activation. Recombinant antibodies against CD55 and CD59 and uses thereof are reported in US 8,034,902. In US 2012/0226020 hybrid and chimeric polypeptides that regulate activation of complement are reported. Novel modulators and methods of use are reported in US 2013/0302355. In US 2010/0255011 compositions and methods for modulating the activity of complement regulatory proteins on target cells are reported.

In WO 2008/007648 it is reported that classifying antibody, involves contacting antibody capable of recognizing cell surface antigen with cell of same species, analyzing each cell and comparing obtained data and classifying individual antibodies depending on similarity. Compositions and methods for modulating the activity of complement regulatory proteins on target cells are reported in WO 2010/120541.

Mekhaiel, D.N.A., et al, report polymeric human Fc-fusion proteins with modified effector functions (Nature Sci. Rep. 1 (2011) 1-11). Polypeptide variants with altered effector function are reported in WO 00/42072. In US 2008/0089892 Fc region variants are reported. Altered antibody Fc regions and uses thereof are reported in WO 2006/105062.

Neonatal rabbit complement was used to deplete lymphocytes from different complex immune cell populations with the help of antibodies to facilitate transplantation (see e.g. Herve, P., et al., Transplant. 39 (1985) 138-143).

Baby Rabbit complement was not successful in eliciting complement dependent cytotoxicity (CDC) in renal cell carcinoma (RCC) using antibodies of murine origin (see e.g. Vessella, R.L., et al., Canc. Res. 45 (1985) 6131-6139).

Rabbit serum could kill human SK-Me128 melanoma cells (non-epithelial = non-carcinoma) by CDC using single and paired murine IgG2a antibodies binding p97 (= melanotransferrin) (see e.g. Hellstroem, I., et al., Int. J. Canc. 31 (1983) 553-555).

Membrane-bound complement regulatory proteins (mCRPs) have a lower expression level on lymphocytes compared to monocytes and neutrophils (see e.g. Nuutila, J., et al., Hum. Immunol. 74 (2013) 522-530).

The up-regulation of mCRPs as an immune escape mechanism is more pronounced on most of the cancer cells than e.g. on lymphomas or melanomas (see e.g. Fishelson, Z., et al., Mol. Immunol. 40 (2003) 109-123).

Antibodies were used to show CDC either in settings with syngeneic serum (e.g. normal human serum (NHS) together with human carcinoma cells and human antibodies) without the CDC-inhibitory influence of mCRPs (see e.g. Dechant et al., 2008, Cancer Research) or with syngeneic serum (e.g. normal human serum (NHS) together with human carcinoma cells and human antibodies) showing a strong mCRP dependent CDC-inhibitory effect that had to be overcome by the siRNA-dependent down regulation of the mCRPs CD46, CD55 and CD59 (see e.g. Mamidi, S., et al., Mol. Onc.7 (2013) 580-594).

Konishi, e., et al. reported the utilization of complement-dependent cytotoxicity to measure low levels of antibodies: application to nonstructural protein 1 in a model of Japanese encephalitis virus (Clin. Vac. Immunol. 15 (2008) 88-94). Klitgaard, J., et al. reported that the combination of two anti-cos monoclonal antibodies synergistically induces complement-dependent cytotoxicity of chronic lymphocytic leukaemia cells (Brit. J. Hematol. 163 (2013) 182-193). Hellstrom, I., et al. reported that monoclonal antibodies to two determinants of melanoma-antigen p97 act synergistically in complement-dependent cytotoxicity (J. Immunol. 127 (1981) 157-160. Maddipatla, S., et al., reported augmented antitumor activity against B-cell lymphoma by a combination of monoclonal antibodies targeting Trail-RI and CD20 (Clin. Cancer Res. 13 (2007) 4556-4564). Huang, J., et al. reported about the protection of xenogeneic cells from human complement-mediated lysis by the expression of human DAF, CD59 and MCP (FEMS Immunol. Med. Microbiol. 31 (2001) 203-209. Qu, Z., et al. reported about recombinant bispecific monoclonal antibody (bsMab) against CD20 and CD22 active in vitro and in vivo against B-cell lymphomas (Blood 108 (2006) 713a-714a).

Konishi, E., et al., disclosed the utilization of Complement-Dependent Cytotoxicity to measure low levels of antibodies by application to nonstructural protein 1 in a model of Japanese Encephalitis Virus (Clin. Vacc. Immunol. 15 (2008) 88-94).

Klitgaard, J.L., et al. disclosed that the combination of two anti-CD5 monoclonal antibodies synergistically induces complement-dependent cytotoxicity of chronic lymphocytic leukaemia cells (Brit. J. Haematol. 163 (2013) 182-193).

Hellstrom, I. et al., disclosed that monoclonal antibodies to two determinants of melanoma-antigen p97 act synergistically in complement-dependent cytotoxicity (J. Immunol. 127 (1981) 157-160).

Maddipatla, S., et al., disclosed augmented antitumor activity against B-cell lymphoma by a combination of monoclonal antibodies targeting TRAIL-R1 and CD20 (Clin. Cancer Res. 13 (2007) 4556-4564).

Huang, J., et al., disclosed the protection of xenogeneic cells from human complement-mediated lysis by the expression of human DAF, CD59 and MCP (FEMS Immunol. Med. Microbiol. 31 (2001) 203-209).

Qu, Z., et al., disclosed recombinant bispecific monoclonal antibody (bsMAb) against CD20 and CD22 active in vitro and in vivo against B-cell lymphomas (Blood 108 (2006) 713A-714A).

## SUMMARY

The invention is defined in the appended claims and any other aspects set forth herein not falling within the scope of the claims are for information only.

Herein is reported an improved assay for the analysis of the CDC capacity regarding carcinoma cells of carcinoma-cell surface antigen binding antibodies. This assay does not require e.g. tedious, complicated and instable approaches, such as e.g. siRNA down-regulation of the mCRPs. This approach counteracts the up-regulation of mCRPs in carcinoma cells as immune escape mechanism evading the CDC pressure in the body. The current assay provides a means to determine CDC of carcinoma-cell surface antigen binding antibodies that cannot elicit CDC in other settings due to the effect of the mCRPs.

It has been found that a non-syngeneic serum like rabbit complement together with human or humanized antibodies and human carcinoma cells can be used to elicit complement dependent cytotoxicity (CDC) in human cells, especially human carcinoma cells, in a very robust manner. By using BRC for the determination of the CDC capacity of human or humanized antibodies specifically binding to carcinoma cell surface antigens

- the up-regulated human mCRPs on carcinoma cells do not abrogate the CDC-eliciting effect of human or humanized antibodies observed in other assay setups,

- the unreliability that in some cases normal human serum (NHS) could only elicit CDC with human or humanized antibodies and human tumor cells, if the mCRPs were down-regulated by siRNA, could be overcome, and

- high throughput screening of the CDC capacity of different antibodies, antibody formats or antibody conjugates is now possible.

The method as reported herein can be used with cancer cells, such as lymphoma cells or carcinoma cell of epithelial origin, as well as cell eliciting an autoimmune response.

One aspect as reported herein is a method for determining complement dependent cytotoxicity of a composition wherein the composition comprises i) a first binding site that specifically binds to a first epitope on a first antigen, which is conjugated to a first Fc-region polypeptide of human origin, and ii) a second binding site that specifically binds to a second epitope on the first antigen, which is conjugated to a second Fc-region polypeptide of human origin, wherein the method comprises the following steps:

a) incubating a human carcinoma cell of epithelial origin expressing the first antigen with the composition,
b) adding rabbit complement to the mixture of a), and
c) determining cell lysis and thereby determining complement dependent cytotoxicity of the composition.

In one embodiment the antibody is an antibody format.

In one embodiment the two or more compositions differ in the first and/or second epitope.

In one embodiment of all aspects the composition comprises a first human or humanized antibody that specifically binds to a first epitope on a first antigen and a second human or humanized antibody that specifically binds to a second epitope on the first antigen.

In one embodiment of all aspects the composition comprises a human or humanized bispecific antibody that specifically binds to a first epitope on a first antigen and a second epitope on the first antigen.

In one embodiment of all aspects the first epitope and the second epitope are different. In one embodiment the first epitope and the second epitope are nonoverlapping epitopes.

In one embodiment of all aspects cell lysis is determined between 0.5 and 3 hours after the addition of complement.

The cell is a human carcinoma cell of epithelial origin.

In one embodiment of all aspects the method is a serum-free method.

In one preferred embodiment of all aspects the rabbit complement is Baby Rabbit complement.

In one embodiment the ratio of the first binding site to the second binding site is of from 10:1 to 1:10. In one preferred embodiment the ratio is of from 0.5:1 to 1:0.5.

## BRIEF DESCRIPTION OF THE FIGURES

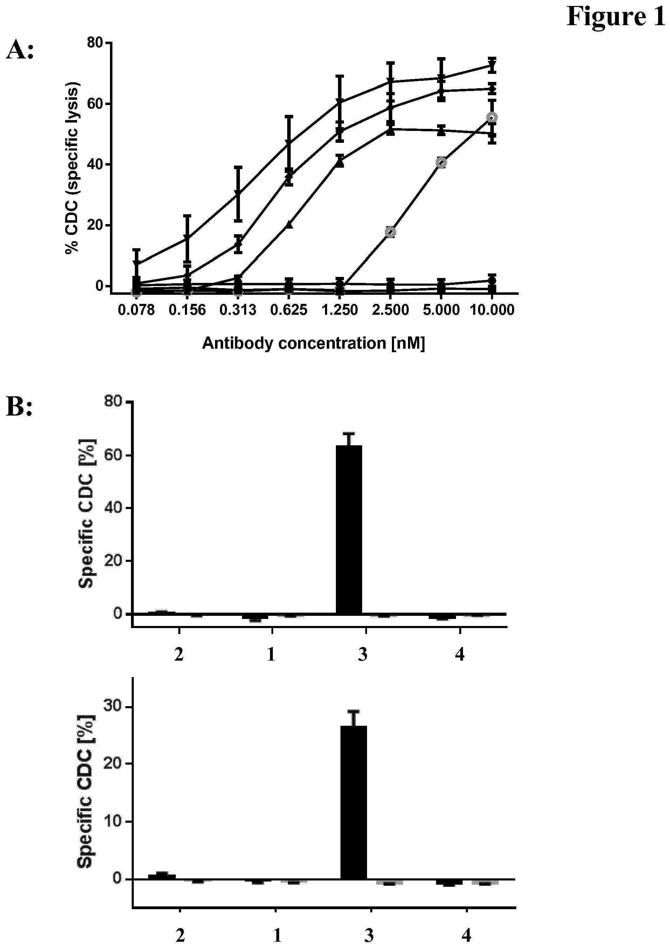

Figure 1: A: Specific CDC on BT-474 cells determined by LDH release and shown as % CDC; closed circles: trastuzumab; closed squares: pertuzumab; upward triangle: combination of trastuzumab and pertuzumab; downward triangle = bispecific anti-HER2 antibody, common light chain; diamond = bispecific anti-HER2 antibody, common light chain, glycoengineered; open circle = bispecific anti-HER2 antibody, CrossMab format.

B: Specific CDC on BT-474 cells (upper graph) and SK-Br3 cells (lower graph); 1 = trastuzumab; 2 = pertuzumab; 3 = combination of trastuzumab and pertuzumab; 4 = human IgG1, kappa light chain control; left bars: specific CDC with Baby Rabbit complement; right bars: specific CDC without Baby Rabbit complement; % CDC and specific CDC means specific cytotoxicity [%].

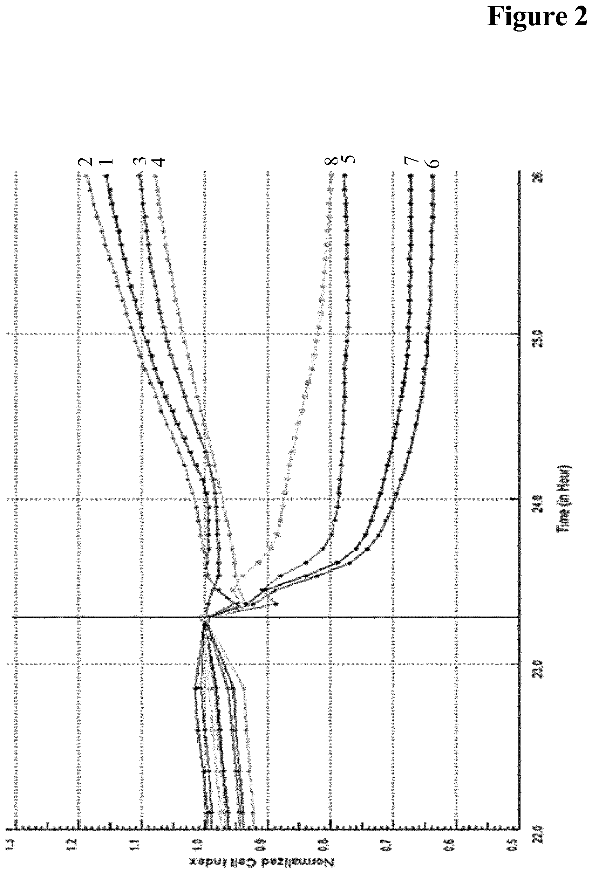

Figure 2: Time course of cell index (ACEA); 1 = trastuzumab; 2 = pertuzumab; 3 = medium only; 4 = complement control; 5 = combination of trastuzumab and Pertuzumab; 6 = bispecific anti-HER2 antibody, common light chain; 7 = bispecific anti-HER2 antibody, common light chain, glycoengineered; 8 = bispecific anti-HER2 antibody, CrossMab format.

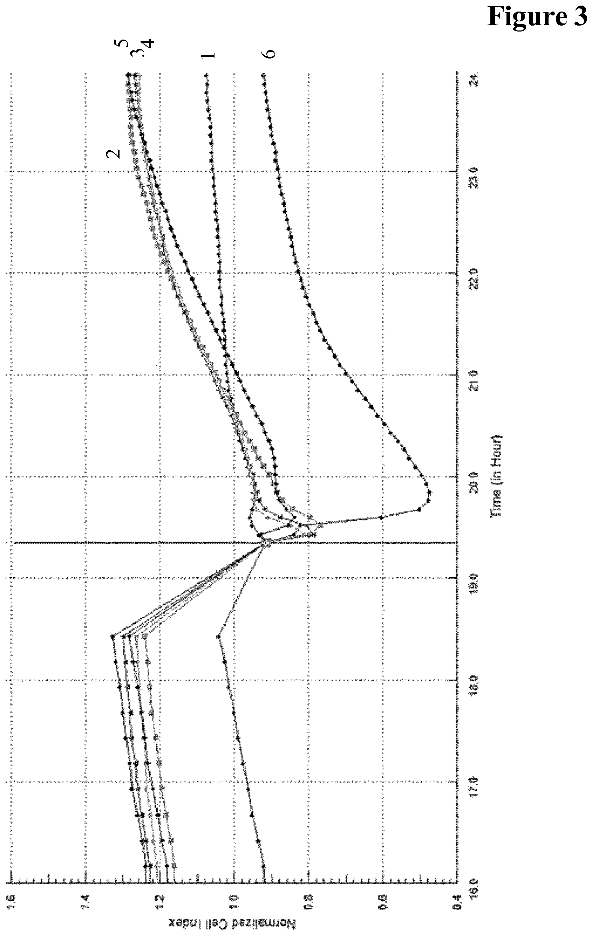

Figure 3: Time course of cell index (ACEA); 1 = medium only; 2 = complement control; 3 = with anti-CD55 antibody, human serum pool, trastuzumab, pertuzumab; 4 = with anti-CD59 antibody, human serum pool, trastuzumab, pertuzumab; 5 = with anti-CD55 antibody, anti-CD59 antibody, human serum pool, trastuzumab and pertuzumab; 6 = trastuzumab, pertuzumab and Baby Rabbit complement.

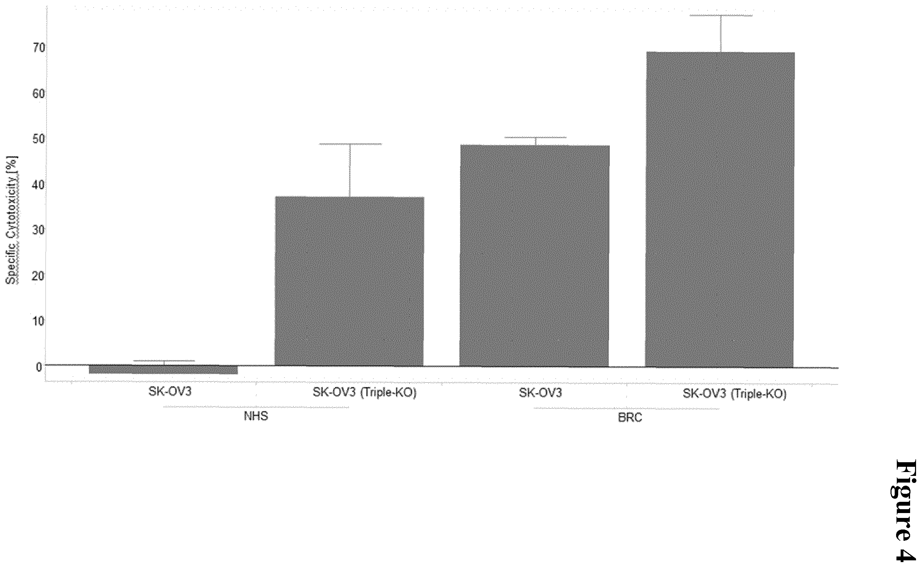

Figure 4: Results of the CDC assay using CD46, CD55, CD59 knockdown (triple-KO) SK-OV-3 cells. Cells were incubated with 10 µg/mL antibody each, Baby Rabbit complement and Normal Human Serum respectively.

## DETAILED DESCRIPTION OF EMBODIMENTS OF THE INVENTION

### I. DEFINITIONS

The term "Clq binding" denotes the binding of Clq to an antibody bound to its antigen. The binding of the antibody to its antigen is without limitation in vivo and in vitro within the methods and assays as reported herein.

In one embodiment Clq binding is determined in a method comprising i) coating a multi-well plate (e.g. a 96-well ELISA plate) overnight at 4°C with antibody in PBS at a concentration ranging from 0.007 to 25.0 mg/mL, ii) washing the plates, iii) blocking remaining reactive surface residues with 0.5 x PBS/0.025 % Tween 20/0.1% gelatin, iv) incubating the multi-well plates for one hour at 37 °C with a) 3 % pooled human serum, b) rabbit anti-human Clq, and c) swine anti-rabbit IgG antibody conjugated to HRP, comprising in-between washing, v) incubating for about 30 min with 1 mg/mL 2,2'-azino-bis 3-ethylbenzothiazoline-6-sulfonic acid, vi) adding 100 µL 2% oxalic acid, and vii) measuring the absorbance at 405 nm in a microplate reader.

C1q binding of an antibody denotes herein a multivalent interaction resulting in high avidity binding.

The term "complement activation" denotes the initiation of the classical complement pathway. This initiation results from the binding of complement component Clq to the antibody-antigen complex. Clq is the first protein in the classical complement cascade. It is involved in a series of reactions that result in the formation of an active C3 convertase, which cleaves complement component C3 into C3b and C3a. C3b binds to membrane C5 resulting in so called C5b which triggers the late events of complement activation (assembly of C5b, C6, C7, C8 and C9 into the membrane attack complex (MAC)). Finally the complement cascade results in the formation of pores in the cell wall causing cell lysis (aka complement dependent cytotoxicity, CDC).

The term "complement-dependent cytotoxicity (CDC)" denotes the process of antibody-mediated complement activation resulting in the lysis of a cell according to the mechanism outlined above upon binding of the antibody to its antigen located on that cell. CDC can be determined in vitro using specific CDC assay. In the art normal human serum is used as a complement source.

The term "complement-dependent cellular cytotoxicity (CDCC)" denotes the process of cell killing mediated by cells expressing complement receptors that recognize complement 3 (C3) cleavage products (located on target cells and resulting from antibody-mediated complement activation).

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant ($k_d$). Affinity can be measured by common methods known in the art, including those described herein. Specific illustrative and exemplary embodiments for measuring binding affinity are described in the following.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity and can elicit CDC.

"Effector functions" refer to those biological activities attributable to the Fc-region of an antibody, which vary with the antibody class. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain which are hypervariable in sequence ("complementarity determining regions" or "CDRs") and form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3).

HVRs herein include

   (a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
   (b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242);
   (c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
   (d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman, S. et al., J. Chromatogr. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The murine monoclonal antibody 4D5 is targeting HER2 specifically in HER2 overexpressing cancer cells, while having no effect on cells expressing physiological levels of HER2. The humanized (4D5) monoclonal antibody (hu4D5) is commercially known as the drug Herceptin® (trastuzumab, rhuMab HER2, US 5,821,337), which gained FDA marketing approval in late 1998.

Pertuzumab (PERJETA™, rhuMab 2C4, US 7,862,817) is a humanized monoclonal antibody, which is designed specifically to prevent the HER2 receptor from pairing (dimerising) with other HER receptors (EGFR/HER1, HER3 and HER4) on the surface of cells, a process that is believed to play a role in tumor growth and survival. PERJETA is approved in combination with Herceptin (trastuzumab) and docetaxel in adult patients with HER2-positive metastatic or locally recurrent non-resectable breast cancer and gained FDA approval for neoadjuvant breast cancer treatment in September 2013.

Pertuzumab binds to domain II of HER2, essential for dimerization, while trastuzumab binds to extracellular domain IV of HER2.

The term "cancer" as used herein refers to proliferative diseases, such as lymphomas, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewing's sarcoma, including refractory versions of any of the above cancers, or a combination of one or more of the above cancers.

The term "antigen-binding site" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

Antibody specificity refers to selective recognition of the antibody for a particular epitope of an antigen. Natural antibodies, for example, are monospecific. The term "monospecific" antibody as used herein denotes an antibody that has one or more binding sites each of which bind to the same epitope of the same antigen.

"Bispecific antibodies" are antibodies which have two different antigen-binding specificities. The term "bispecific" antibody as used herein denotes an antibody that has at least two binding sites each of which bind to different epitopes.

The term "valent" as used within the current application denotes the presence of a specified number of binding sites in an antibody molecule. As such, the terms "bivalent", "tetravalent", and "hexavalent" denote the presence of two binding sites, four binding sites, and six binding sites, respectively, in an antibody molecule. The bispecific antibodies according to the invention are at least "bivalent" and may be "trivalent" or "multivalent" (e.g. "tetravalent" or "hexavalent").

As used herein, the term "binding" or "specifically binding" refers to the binding of the antibody to an epitope of the antigen in an in-vitro assay, preferably in a surface plasmon resonance assay (SPR, BIAcore, GE-Healthcare Uppsala, Sweden). The affinity of the binding is defined by the terms ka (rate constant for the association of the antibody from the antibody/antigen complex), $k_d$ (dissociation constant), and $K_D$ ($k_d/k_a$). Binding or specifically binding means a binding affinity ($K_D$) of $10^{-7}$ mol/L or less.

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

## II. METHODS AS REPORTED HEREIN

Carcinomas are of epithelial origin and the cells often upregulate the mCRPs (especially CD55 and CD59) as immune escape mechanism evading the CDC pressure in vivo. In some cases the carcinoma-cell surface antigen binding antibodies cannot elicit CDC due to the effect/presence of the mCRPs. In the past this has been addressed in carcinoma cells using tedious, complicated and instable approaches, such as e.g. siRNA down-regulation of the mCRPs. Herein is reported an improved, i.e. among other things more robust and high-throughput compatible, assay for the analysis of the CDC capacity of carcinoma-cell surface antigen binding antibodies.

It has been found that for determining complement dependent cytotoxicity of a composition that comprises molecules that on the one hand specifically bind to one or more cell surface antigens and that on the other hand comprise an Fc-region polypeptide of human origin, e.g. a combination of two or more human or humanized antibodies or a human or humanized bispecific antibody, a non-syngeneic complement, i.e. rabbit complement, e.g. Baby Rabbit complement, has to be used. Unexpectedly the use of syngeneic complement did not result in a functional method. Likewise the use of Guinea pig complement (a specific non-syngeneic complement) also did not result in a functional assay.

Herein is reported a method to determine the CDC-activity of antibody combinations or of bispecific antibodies. The method is especially useful in cases in which the incubation with human serum and human cancer cells does not provide for a reliable result.

One aspect as reported herein is a method for determining complement dependent cytotoxicity of a composition comprising i) a first binding site that specifically binds to a first epitope on a first antigen, which is conjugated to a first Fc-region polypeptide of human origin, and ii) a second binding site that specifically binds to a second epitope on the first antigen, which is conjugated to a second Fc-region polypeptide of human origin,wherein the method comprises the following steps: a) incubating a human carcinoma cell of epithelial origin expressing the first antigen with the composition, b) adding rabbit complement to the mixture of a), and c) determining cell lysis and thereby determining complement dependent cytotoxicity of the composition.

It has been found that monospecific antibodies do not work in the assay as reported herein.

It has been surprisingly been found that the combination of human cancer cells, a human or humanized antibody and a non-syngeneic complement of rabbit origin results in a functional assay.

In one embodiment the cell expresses the first epitope and the second epitope.

In one embodiment the first antigen is a cell surface antigen.

The cell expressing the cell surface antigens is a carcinoma cell.

Complement dependent cytotoxicity should be determined one or two hours after the addition of complement. Thus, in one embodiment cell lysis is determined between 0.5 hours and 3 hours after the addition of complement, i.e. of Baby Rabbit complement. In one embodiment cell lysis is determined between 1 hour and 2 hours after the addition of complement.

Cell lysis can be determined with any suitable method, such as e.g. LDH release or cell viability determination. Thus, in one embodiment cell lysis is determined by determining LDH release or cell viability.

The method as reported herein does not need the presence of serum. Thus, in one embodiment the method is a serum free method.

The method as reported herein can be used for the selection of antibody combinations which do not cross-compete with each other for binding but to exert CDC in combination (not alone).

One aspect as reported herein is a method for determining complement dependent cytotoxicity of a composition

wherein the composition comprises

i) a first binding site that specifically binds to a first epitope on a first antigen, which is conjugated to a first Fc-region polypeptide of human origin, and
ii) a second binding site that specifically binds to a second epitope on the first antigen, which is conjugated to a second Fc-region polypeptide of human origin,

wherein the method comprises the following steps:

a) incubating a human cell expressing the first antigen with the composition,
b) adding rabbit complement to the mixture of a), and
c) determining cell lysis and thereby determining complement dependent cytotoxicity of the composition.

One aspect as reported herein is a method for determining complement dependent cytotoxicity of a combination of two monospecific antibodies or of a bispecific antibody

wherein

i) the first monospecific antibody specifically binds to a first epitope on a first antigen, and the second monospecific antibody specifically binds to a second epitope on the first antigen, or
ii) the bispecific antibody comprises a first binding site that specifically binds to a first epitope on a first antigen, and a second binding site that specifically binds to a second epitope on the first antigen,

wherein the method comprises the following steps:

a) incubating a human carcinoma cell of epithelial origin expressing the first antigen with the combination of the two monospecific antibodies or with the bispecific antibody,
b) adding rabbit complement to the mixture of a), and
c) determining cell lysis and thereby determining complement dependent cytotoxicity of the combination of two monospecific antibodies or of the bispecific antibody.

## Humanized Antibodies

Typically, a non-human antibody that is intended to be used as therapeutic is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of or a full length human constant region. In some embodiments, some FR residues in a humanized antibody are substituted with corresponding residues from a non-human antibody (e.g. the antibody from which the HVR residues are derived), e.g. to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I. et al., Nature 332 (1988) 323-329; Queen, C. et al., Proc. Natl. Acad. Sci. USA 86 (1989) 10029-10033; US 5,821,337, US 7,527,791, US 6,982,321, and US 7,087,409; Kashmiri, S.V., et al., Methods 36 (2005) 25-34 (describing specificity determining region (SDR) grafting); Padlan, E.A., Mol. Immunol. 28 (1991) 489-498 (describing "resurfacing"); Dall'Acqua, W.F., et al., Methods 36 (2005) 43-60 (describing "FR shuffling"); Osbourn, J., et al., Methods 36 (2005) 61-68; and Klimka, A., et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J., et al., J. Immunol. 151 (1993) 2296-2308; framework regions derived from the consensus sequence of human antibodies of a particular subgroup of light or heavy chain variable regions (see, e.g., Carter, P., et al., Proc. Natl. Acad. Sci. USA 89 (1992) 4285-4289; and Presta, L.G., et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M., et al., J. Biol. Chem. 272 (1997) 10678-10684; and Rosok, M.J., et al., J. Biol. Chem. 271 (19969 22611-22618).

## Multispecific Antibodies

In certain embodiments, an antibody used in the method reported herein is a multispecific antibody, e.g. a bispecific antibody. Multispecific antibodies are monoclonal antibodies that have binding specificities for at least two different sites/antigens/epitopes. In certain embodiments, bispecific antibodies may bind to two different epitopes of the same antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A., et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (see WO 2009/089004); crosslinking two or more antibodies or fragments (see, e.g., US 4,676,980, and Brennan, M., et al., Science 229 (1985) 81-83); using leucine zippers to produce bi-specific antibodies (see, e.g., Kostelny, S.A., et al., J. Immunol. 148 (1992) 1547-1553); using "diabody" technology for making bispecific antibody fragments (see, e.g., Holliger, P., et al., Proc. Natl. Acad. Sci. USA 90 (1993) 6444-6448); using single-chain Fv (sFv) dimers (see, e.g. Gruber, M., et al., J. Immunol. 152 (1994) 5368-5374); and preparing trispecific antibodies as described, e.g., in Tutt, A., et al., J. Immunol. 147 (1991) 60-69).

Engineered antibodies with three or more functional antigen binding sites, including "Octopus antibodies," are also included herein (see, e.g. US 2006/0025576).

The antibody also includes a "Dual Acting Fab" or "DAF" (see, US 2008/0069820, for example).

The antibody or fragment herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793.

## Recombinant Methods and Compositions

Antibodies may be produced using recombinant methods and compositions, e.g., as described in US 4,816,567. For expression nucleic acids encoding the individual polypeptide chains of the antibody are required. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody, the nucleic acid(s) encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523; see also Charlton, K.A., In: Methods in Molecular Biology, Vol. 248, Lo, B.K.C. (ed.), Humana Press, Totowa, NJ (2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern (see Gerngross, T.U., Nat. Biotech. 22 (2004) 1409-1414; and Li, H., et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts (see, e.g., US 5,959,177, US 6,040,498, US 6,420,548, US 7,125,978, and US 6,417,429 (describing PLANTIBODIES™ technology for producing antibodies in transgenic plants)). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L., et al., J. Gen Virol. 36 (1977) 59-74); baby hamster kidney cells (BHK); mouse sertoli cells (TM4 cells as described, e.g., in Mather, J.P., Biol.

Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P., et al., Annals N.Y. Acad. Sci. 383 (1982) 44-68; MRC 5 cells; and FS4 cells. Other useful mammalian host cell lines include Chinese hamster ovary (CHO) cells, including DHFR$^-$ CHO cells (Urlaub, G., et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), 255-268.

**Pharmaceutical Formulations**

Pharmaceutical formulations of antibodies are prepared by mixing such antibodies having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) peptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US 2005/0260186 and US 2006/0104968.

Exemplary lyophilized antibody formulations are described in US 6,267,958. Aqueous antibody formulations include those described in US 6,171,586 and WO 2006/044908, the latter formulations including a histidine-acetate buffer.

The formulation may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

**Therapeutic Methods and Compositions**

Any of the compositions, i.e. antibody combinations or multispecific antibodies, selected with a method provided herein may be used in therapeutic methods.

In one aspect, a composition selected with a method as reported herein for use as a medicament is provided. In certain embodiments, a composition selected with a method as reported herein for use in a method of treatment is provided. In certain embodiments, the invention provides a composition selected with a method as reported herein for use in a method of treating an individual comprising administering to the individual an effective amount of the composition selected with a method as reported herein. In one such embodiment, the method further comprises administering to the individual an effective amount of at least one additional therapeutic agent. An "individual" according to any of the above embodiments is preferably a human.

In a further aspect, the invention provides for the use of a composition selected with a method as reported herein in the manufacture or preparation of a medicament. In a further embodiment, the composition selected with a method as reported herein is for use in a method of treating a disease comprising administering to an individual having the disease an effective amount of the composition selected with a method as reported herein. In one such embodiment, the method further comprises administering to the individual an effective amount of at least one additional therapeutic agent. An "individual" according to any of the above embodiments may be a human.

In a further aspect, the invention provides pharmaceutical formulations comprising a composition selected with a method as reported herein, e.g., for use in any of the above therapeutic methods. In one embodiment, a pharmaceutical formulation comprises any of the compositions selected with a method as reported herein and a pharmaceutically acceptable carrier. In another embodiment, a pharmaceutical formulation comprises any of the compositions selected with a method as reported herein and at least one additional therapeutic agent.

Compositions selected with a method as reported herein can be used either alone or in combination with other agents in a therapy. For instance, a composition selected with a method as reported herein may be co-administered with at least one additional therapeutic agent.

Such combination therapies noted above encompass combined administration (where two or more therapeutic agents are included in the same or separate formulations), and separate administration, in which case, administration of the composition selected with a method as reported herein can occur prior to, simultaneously, and/or following, administration of the additional therapeutic agent or agents. In one embodiment, administration of the composition selected with a method as reported herein and administration of an additional therapeutic agent occur within about one month, or within about one, two or three weeks, or within about one, two, three, four, five, or six days, of each other.

A composition selected with a method as reported herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Compositions selected with a method as reported herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The composition selected with a method as reported herein need not be, but is optionally formulated with one or more agents currently used to prevent or treat the disorder in question. The effective amount of such other agents depends on the amount of the components present in the formulation, the type of disorder or treatment, and other factors discussed above. These are generally used in the same dosages and with administration routes as described herein, or about from 1 to 99 % of the dosages described herein, or in any dosage and by any route that is empirically/clinically determined to be appropriate.

For the prevention or treatment of disease, the appropriate dosage of a composition selected with a method as reported herein (when used alone or in combination with one or more other additional therapeutic agents) will depend on the type of disease to be treated, the type of composition, the severity and course of the disease, whether the composition is administered for preventive or therapeutic purposes, previous therapy, the patient's clinical history and response to the composition, and the discretion of the attending physician. The composition selected with a method as reported herein is suitably administered to the patient at one time or over a series of treatments. Depending on the type and severity of the disease, about 1 $\mu$g/kg to 15 mg/kg (e.g. 0.5 mg/kg - 10 mg/kg) of composition can be an initial candidate dosage for administration to the patient, whether, for example, by one or more separate administrations, or by continuous infusion. One typical daily dosage might range from about 1 $\mu$g/kg to 100 mg/kg or more, depending on the factors mentioned above. For repeated administrations over several days or longer, depending on the condition, the treatment would generally be sustained until a desired suppression of disease symptoms occurs. One exemplary dosage of the composition would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

## III. EXAMPLES

The following are examples of methods and compositions of the invention. It is understood that various other embodiments may be practiced, given the general description provided above.

**Materials and Methods**

Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

Gene and oligonucleotide synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH

(Planegg-Martinsried, Germany)

Reagents

All commercial chemicals, antibodies and kits were used as provided according to the manufacturer's protocol if not stated otherwise.

Antibodies

trastuzumab

light chain:

```
DIQMTQSPSS LSASVGDRVT ITCRASQDVN TAVAWYQQKP
GKAPKLLIYS ASFLYSGVPS RFSGSRSGTD FTLTISSLQP
EDFATYYCQQ HYTTPPTFGQ GTKVEIKRTV AAPSVFIFPP
SDEQLKSGTA SVVCLLNNFY PREAKVQWKV DNALQSGNSQ
ESVTEQDSKD STYSLSSTLT LSKADYEKHK VYACEVTHQG
LSSPVTKSFN RGEC (SEQ ID NO: 01)
```

heavy chain:

```
EVQLVESGGG LVQPGGSLRL SCAASGFNIK DTYIHWVRQA
PGKGLEWVAR IYPTNGYTRY ADSVKGRFTI SADTSKNTAY
LQMNSLRAED TAVYYCSRWG GDGFYAMDYW GQGTLVTVSS
ASTKGPSVFP LAPSSKSTSG GTAALGCLVK DYFPEPVTVS
WNSGALTSGV HTFPAVLQSS GLYSLSSVVT VPSSSLGTQT
YICNVNHKPS NTKVDKKVEP KSCDKTHTCP PCPAPELLGG
PSVFLFPPKP KDTLMISRTP EVTCVVVDVS HEDPEVKFNW
YVDGVEVHNA KTKPREEQYN STYRVVSVLT VLHQDWLNGK
EYKCKVSNKA LPAPIEKTIS KAKGQPREPQ VYTLPPSREE
MTKNQVSLTC LVKGFYPSDI AVEWESNGQP ENNYKTTPPV
LDSDGSFFLY SKLTVDKSRW QQGNVFSCSV MHEALHNHYT
QKSLSLSPGK (SEQ ID NO: 02)
```

pertuzumab

light chain:

```
DIQMTQSPSS LSASVGDRVT ITCKASQDVS IGVAWYQQKP
GKAPKLLIYS ASYRYTGVPS RFSGSGSGTD FTLTISSLQP
EDFATYYCQQ YYIYPYTFGQ GTKVEIKRTV AAPSVFIFPP
SDEQLKSGTA SVVCLLNNFY PREAKVQWKV DNALQSGNSQ
ESVTEQDSKD STYSLSSTLT LSKADYEKHK VYACEVTHQG
LSSPVTKSFN RGEC (SEQ ID NO: 03)
```

heavy chain:

```
EVQLVESGGG LVQPGGSLRL SCAASGFTFT DYTMDWVRQA
PGKGLEWVAD VNPNSGGSIY NQRFKGRFTL SVDRSKNTLY
LQMNSLRAED TAVYYCARNL GPSFYFDYWG QGTLVTVSSA
STKGPSVFPL APSSKSTSGG TAALGCLVKD YFPEPVTVSW
NSGALTSGVH TFPAVLQSSG LYSLSSVVTV PSSSLGTQTY
ICNVNHKPSN TKVDKKVEPK SCDKTHTCPP CPAPELLGGP
SVFLFPPKPK DTLMISRTPE VTCVVVDVSH EDPEVKFNWY
VDGVEVHNAK TKPREEQYNS TYRVVSVLTV LHQDWLNGKE
YKCKVSNKAL PAPIEKTISK AKGQPREPQV YTLPPSRDEL
TKNQVSLTCL VKGFYPSDIA VEWESNGQPE NNYKTTPPVL
DSDGSFFLYS KLTVDKSRWQ QGNVFSCSVM HEALHNHYTQ
KSLSLSPGK (SEQ ID NO: 04)
```

bispecific anti-HER2 antibody, common light chain

common light chain:

```
DIQMTQSPSS LSASVGDRVT ITCKASQDVS TAVAWYQQKP
GKAPKLLIYS ASFRYTGVPS RFSGSRSGTD FTLTISSLQP
EDFATYYCQQ HYTTPPTFGQ GTKVEIKRTV AAPSVFIFPP
SDEQLKSGTA SVVCLLNNFY PREAKVQWKV DNALQSGNSQ
ESVTEQDSKD STYSLSSTLT LSKADYEKHK VYACEVTHQG
LSSPVTKSFN RGEC (SEQ ID NO: 05)
```

heavy chain 1 (knob, trastuzumab):

```
EVQLVESGGG LVQPGGSLRL SCAASGFNIK DTYIHWVRQA
PGKGLEWVAR IYPTNGYTRY ADSVKGRFTI SADTSKNTAY
LQMNSLRAED TAVYYCSRWG GDGFYAMDYW GQGTLVTVSS
ASTKGPSVFP LAPSSKSTSG GTAALGCLVK DYFPEPVTVS
WNSGALTSGV HTFPAVLQSS GLYSLSSVVT VPSSSLGTQT
YICNVNHKPS NTKVDKKVEP KSCDKTHTCP PCPAPELLGG
PSVFLFPPKP KDTLMISRTP EVTCVVVDVS HEDPEVKFNW
YVDGVEVHNA KTKPREEQYN STYRVVSVLT VLHQDWLNGK
EYKCKVSNKA LPAPIEKTIS KAKGQPREPQ VYTLPPCRDE
LTKNQVSLWC LVKGFYPSDI AVEWESNGQP ENNYKTTPPV
LDSDGSFFLY SKLTVDKSRW QQGNVFSCSV MHEALHNHYT
QKSLSLSPGK (SEQ ID NO: 06)
```

heavy chain 2 (hole, pertuzumab):

```
EVQLVESGGG LVQPGGSLRL SCAASGFTFN DYTMDWVRQA
PGKGLEWVAD VNPNSGGSIV NRRFKGRFTL SVDRSKNTLY
LQMNSLRAED TAVYYCARNL GPFFYFDYWG QGTLVTVSSA
STKGPSVFPL APSSKSTSGG TAALGCLVKD YFPEPVTVSW
NSGALTSGVH TFPAVLQSSG LYSLSSVVTV PSSSLGTQTY
ICNVNHKPSN TKVDKKVEPK SCDKTHTCPP CPAPELLGGP
SVFLFPPKPK DTLMISRTPE VTCVVVDVSH EDPEVKFNWY

VDGVEVHNAK TKPREEQYNS TYRVVSVLTV LHQDWLNGKE
YKCKVSNKAL PAPIEKTISK AKGQPREPQV CTLPPSRDEL
TKNQVSLSCA VKGFYPSDIA VEWESNGQPE NNYKTTPPVL
DSDGSFFLVS KLTVDKSRWQ QGNVFSCSVM HEALHNHYTQ
KSLSLSPGK (SEQ ID NO: 07)
```

bispecific anti-HER2 antibody, CrossMab format:

heavy chain 1:

```
QVQLVQSGAE VKKPGASVKV SCKASGFNIK DTYIHWVRQA
PGQGLEWMGR IYPTNGYTRY AQKFQGRVTM TRDTSISTAY
MELSRLRSDD TAVYYCSRWG GEGFYAMDYW GQGTMVTVSS
ASTKGPSVFP LAPSSKSTSG GTAALGCLVK DYFPEPVTVS
WNSGALTSGV HTFPAVLQSS GLYSLSSVVT VPSSSLGTQT
YICNVNHKPS NTKVDKKVEP KSCDKTHTCP PCPAPELLGG
PSVFLFPPKP KDTLMISRTP EVTCVVVDVS HEDPEVKFNW
YVDGVEVHNA KTKPREEQYN STYRVVSVLT VLHQDWLNGK
EYKCKVSNKA LPAPIEKTIS KAKGQPREPQ VCTLPPSRDE
LTKNQVSLSC AVKGFYPSDI AVEWESNGQP ENNYKTTPPV
LDSDGSFFLV SKLTVDKSRW QQGNVFSCSV MHEALHNHYT
QKSLSLSPGK (SEQ ID NO: 08)
```

heavy chain 2:

```
EVQLVESGGG LVQPGGSLRL SCAASGFTFT DYTMDWVRQA
PGKGLEWVAD VNPNSGGSIY NQRFKGRFTL SVDRSKNTLY
LQMNSLRAED TAVYYCARNL GPSFYFDYWG QGTLVTVSSA
SVAAPSVFIF PPSDEQLKSG TASVVCLLNN FYPREAKVQW
KVDNALQSGN SQESVTEQDS KDSTYSLSST LTLSKADYEK
HKVYACEVTH QGLSSPVTKS FNRGECDKTH TCPPCPAPEL
LGGPSVFLFP PKPKDTLMIS RTPEVTCVVV DVSHEDPEVK
FNWYVDGVEV HNAKTKPREE QYNSTYRVVS VLTVLHQDWL
NGKEYKCKVS NKALPAPIEK TISKAKGQPR EPQVYTLPPC
RDELTKNQVS LWCLVKGFYP SDIAVEWESN GQPENNYKTT
PPVLDSDGSF FLYSKLTVDK SRWQQGNVFS CSVMHEALHN
HYTQKSLSLS PGK (SEQ ID NO: 09)
```

light chain 1:

```
DIQLTQPPSV SVAPGQTARI TCGASQDVST AVAWYQQKPG
QAPVLVVYSA SFLYSGIPSR FSGSRSGTDF TLTISRVEAG
DEADYYCQQH YTTPPTFGTG TKVTVLRTVA APSVFIFPPS
DEQLKSGTAS VVCLLNNFYP REAKVQWKVD NALQSGNSQE
SVTEQDSKDS TYSLSSTLTL SKADYEKHKV YACEVTHQGL
SSPVTKSFNR GEC (SEQ ID NO: 10)
```

light chain 2:

```
DIQMTQSPSS LSASVGDRVT ITCKASQDVS IGVAWYQQKP
GKAPKLLIYS ASYRYTGVPS RFSGSGSGTD FTLTISSLQP
EDFATYYCQQ YYIYPYTFGQ GTKVEIKSSA STKGPSVFPL
APSSKSTGG TAALGCLVKD YFPEPVTVSW NSGALTSGVH
TFPAVLQSSG LYSLSSVVTV PSSSLGTQTY ICNVNHKPSN
TKVDKKVEPK SC (SEQ ID NO: 11)
```

Expression

a) Construction of the expression plasmids

The following expression vector was used for the construction of all heavy and light chain encoding expression plasmids. The vector is composed of the following elements:

- a hygromycin resistance gene as a selection marker,
- an origin of replication, oriP, of Epstein-Barr virus (EBV),
- an origin of replication from the vector pUC18 which allows replication of this plasmid in E. coli,
- a beta-lactamase gene which confers ampicillin resistance in E. coli,
- the immediate early enhancer and promoter from the human cytomegalovirus (HCMV),
- the human immunoglobulin polyadenylation ("poly A") signal sequence.

The immunoglobulin genes comprising the heavy or light chain were prepared by gene synthesis and cloned into pGA18 (ampR) plasmids as described above. Variable heavy chain constructs were constructed by directional cloning using unique restriction sites. Variable light chain constructs were ordered as gene synthesis comprising VL and CL and constructed by directional cloning using unique restriction sites. The final expression vectors were transformed into E. coli cells, expression plasmid DNA was isolated (Miniprep) and subjected to restriction enzyme analysis and DNA sequencing. Correct clones were grown in 150 ml LB-Amp medium, again plasmid DNA was isolated (Maxiprep) and sequence integrity confirmed by DNA sequencing.

b) Transient expression of immunoglobulin variants in HEK293 cells

Recombinant immunoglobulins were expressed by transient transfection of human embryonic kidney 293-F cells using the FreeStyle™ 293 Expression System according to the manufacturer's instruction (Invitrogen, USA). For small scale test expressions 30 mL of 0.5 x $10^6$ HEK293F cells/mL were seeded one day prior to transfection. The next day, plasmid DNA (1 μg DNA per mL culture volume) was mixed with 1.2 mL Opti-MEM® I Reduced Serum Medium (Invitrogen, Carlsbad, CA, USA) followed by addition of 40 μL of 293Fectin™ Transfection Reagent (Invitrogen, Carlsbad, CA, USA). The mixture was incubated for 15 min. at room temperature and added drop wise to the cells. One day post-transfection each flask was fed with 300 μL L-glutamine (200 mM, Sigma-Aldrich, Steinheim, Germany) and 600 μL of a feed containing amino acids, sugar, trace elements, FreeStyle medium without RPMI. Three days post-transfection cell concentration, viability and glucose concentration in the medium were determined using an automated cell viability analyzer (Vi-CELL™ XR, Beckman Coulter, Fullerton, CA, USA) and a glucose meter (Accu-CHEK® Sensor comfort, Roche Diagnostics GmbH, Mannheim, Germany). In addition each flask was fed with 300 μL of L-glutamine, 300 μL non-essential amino acids solution (PANTM Biotech, Aidenbach, Germany), 300 μL sodium pyruvate (100 mM, Gibco, Invitrogen), 1.2 ml feed and ad 5 g/L glucose (D-(+)-glucose solution 45 %, Sigma). Finally, six days post-transfection antibodies were harvested by centrifugation at 3500 rpm in a X3R Multifuge (Heraeus, Buckinghamshire, England) for 15 min. at ambient temperature, the supernatant was sterile filtered through a Steriflip filter unit (0.22 μm Millipore Express PLUS PES membrane, Millipore, Bedford, MA) and stored at -20 °C until further use. Large scale transfections up to 5 L were scaled linearly.

c) Purification

Bispecific antibodies were purified from cell culture supernatants by affinity chromatography using Protein A-Sepharose™ (GE Healthcare, Sweden) and Superdex200 size exclusion chromatography. Briefly, sterile filtered cell culture supernatants were applied on a HiTrap Protein A HP (5 mL) column equilibrated with PBS buffer (10 mM $Na_2HPO_4$, 1 mM $KH_2PO_4$, 137 mM NaCl and 2.7 mM KCl, pH 7.4). Unbound proteins were washed out with equilibration buffer. Antibody and antibody variants were eluted with 0.1 M citrate buffer, pH 2.8, and the protein containing fractions were neutralized with 0.1 mL 1 M Tris, pH 8.5. Eluted protein fractions were pooled, concentrated with an Amicon Ultra centrifugal filter device (MWCO: 30 K, Millipore) to a volume of 3 mL and loaded on a Superdex200 HiLoad 120 mL 16/60 gel filtration column (GE Healthcare, Sweden) equilibrated with 20 mM histidine, 140 mM NaCl, pH 6.0. Fractions containing purified bispecific and control antibodies with less than 5 % high molecular weight aggregates were pooled and stored as 1.0 mg/mL aliquots at -80°C.

d) Protein Quantification

Proteins were quantified by affinity chromatography using the automated Ultimate 3000 system (Dionex, Idstein, Germany) with a pre-packed Poros® A Protein A column (Applied Biosystems, Foster City, CA, USA). All samples were loaded in buffer A (0.2 M $Na_2HPO_4$•[$2H_2O$], pH 7.4) and eluted in buffer B (0.1 M citric acid, 0.2 M NaCl, pH 2.5). In order to determine the protein concentration an extinction coefficient of 1.62 was used for all samples.

e) Analysis of purified proteins

The protein concentration of purified protein samples was determined by measuring the optical density (OD) at 280 nm, using the molar extinction coefficient calculated on the basis of the amino acid sequence. Purity and molecular weight of bispecific and control antibodies were analyzed by SDS-PAGE in the presence and absence of a reducing agent (5 mM 1,4-dithiotreitol) and staining with Coomassie brilliant blue. The NuPAGE® Pre-Cast gel system (Invitrogen, USA) was used according to the manufacturer's instruction (4-20 % Tris-Glycine gels). The aggregate content of bispecific and control antibody samples was analyzed by high-performance SEC using a Superdex 200 analytical size-exclusion column (GE Healthcare, Sweden) in 200 mM $KH_2PO_4$, 250 mM KCl, pH 7.0 running buffer at 25 °C. 25 $\mu$g protein were injected on the column at a flow rate of 0.5 mL/min and eluted isocratic over 50 minutes. Integrity of the amino acid backbone of reduced bispecific antibody light and heavy chains was verified by NanoElectrospray Q-TOF mass spectrometry after removal of N-glycans by enzymatic treatment with Peptide-N-Glycosidase F (Roche Molecular Biochemicals).

f) Analytical HPLC

Antibodies were analyzed using a Agilent HPLC 1100 (Agilent Technologies, Palo Alto, CA, USA) with a TSK-GEL G3000SW gel filtration column (7.5 mm ID x 30 cm, Tosohaas Corp., Montgomeryville, PA, USA). 18 $\mu$L of the eluted proteins were loaded onto the column in Buffer A (0.05 M $K_2HPO_4$/$KH_2PO_4$ in 300 mM NaCl, pH 7.5) and separated based on size.

## Example 1

### Assay using different complement sources

Alamar Blue assay with Guinea Pig complement (GPC)

CHO-K1 Nxre19 cells (IL15R transfected CHO-K1) were seeded at 20,000 cells/well on 96-well flat bottom cell culture plates (NUNC, 100 $\mu$L/well) in DMEM/F12 medium supplemented with GlutaMax (Gibco, Cat. No. 31331-028). Twenty-five microliter of IL15-Fc fusion polypeptide (6-fold end-concentration) were added and incubated for one hour. Thereafter 25 $\mu$L of Guinea Pig complement (Sigma Aldrich, Cat. No. S1639) was added and incubated for 3.5 hours. Afterwards 50 $\mu$L of Alamar Blue (Promega) was added and incubated overnight at 37 °C/5 % $CO_2$. The plates were measured at a wavelength of 550 nm (excitation) and 595 nm (emission).

| sample | signal [AU] | variation coefficient |
|---|---|---|
| cells only | 16290 | 240 |
| 2.5 µg/mL IL15-Fc-fusion without GPC | 16408 | 161 |
| complement only without IL15-Fc-fusion | 4893 | 207 |
| 2.5 µg/ml IL15-Fc-fusion with GPC | 4410 | 360 |
| 1.25 µg/ml IL 15-Fc-fusion with GPC | 4104 | 163 |
| 0.625 µg/ml IL15-Fc-fusion with GPC | 4397 | 299 |
| 0.3125 µg/ml IL15-Fc-fusion with GPC | 4070 | 104 |
| 0.156 µg/ml IL15-Fc-fusion with GPC | 3944 | 198 |
| 0.078 µg/ml IL15-Fc-fusion with GPC | 3817 | 117 |
| 0.039 µg/ml IL15-Fc-fusion with GPC | 4047 | 29 |
| 0.020 µg/ml IL15-Fc-fusion with GPC | 4432 | 293 |
| 0.010 µg/ml IL15-Fc-fusion with GPC | 4381 | 293 |
| 0.005 µg/ml IL15-Fc-fusion with GPC | 4092 | 89 |

From the data it can be seen that Guinea pig complement is toxic at all dilutions even in the absence of Fc-region.

LDH assay with Human complement (HUC)

CHO-K1 Nxre19 cells (IL15R transfected CHO-K1) were seeded at 10,000 cells/well on 96-well flat bottom cell culture plates (NUNC, 100 µL/well) and cultivated overnight in DMEM/F12 medium supplemented with GlutaMax (Gibco, Cat. No. 31331-028). IL15-Fc fusion polypeptide was added (25 µL/well in 5-fold end-concentration) and incubated for one hour. Growth medium was removed and cells were washed once with serum-free medium. Thereafter 190 µL/well serum-free medium and 10 µL of Human complement (Sigma Aldrich, Cat. No. S1764, c = 1 mg/mL) was added. After four hours plates were centrifuged at 200 g and 100 µL/well were transferred to another 96-well flat bottom plate. Thereafter 100 µL of LDH reaction mix (Cytotoxicity Detection Kit, Roche Diagnostics GmbH, Mannheim, Germany) were added. After an incubation of 20 min. at 37 °C the optical density (OD) was measured at 492/690nm on a Tecan Sunrise reader.

| sample | signal [OD] | |
|---|---|---|
| | experiment 1 | experiment 2 |
| 1000 ng/ml IL15-Fc-fusion with HUC | 29.1 | 42.6 |
| 333.3 ng/ml IL15-Fc-fusion with HUC | 32.9 | 42.8 |
| 111.1 ng/ml IL15-Fc-fusion with HUC | 34.0 | 43.1 |
| 37.04 ng/ml IL15-Fc-fusion with HUC | 35.5 | 39.6 |
| 12.35 ng/ml IL15-Fc-fusion with HUC | 37.0 | 39.0 |
| 4.12 ng/ml IL15-Fc-fusion with HUC | 38.4 | 40.7 |
| 1.37 ng/ml IL15-Fc-fusion with HUC | 37.2 | 42.2 |
| 0.46 ng/ml IL15-Fc-fusion with HUC | 29.9 | 32.7 |
| 0 ng/ml IL15-Fc-fusion with HUC | 27.7 | 27.7 |

From the data above it can be seen that Human complement does not exert a dose dependent complement dependent toxicity.

LDH assay with Baby Rabbit complement (BRC)

CHO-K1 Nxre19 cells (IL15R transfected CHO-K1) were seeded at 10,000 cells/well on 96-well flat bottom cell culture plates (NUNC, 100 μL/well) and cultivated overnight in DMEM/F12 medium supplemented with GlutaMax (Gibco, Cat. No. 31331-028). IL15-Fc fusion polypeptide was added (25 μL/well in 5-fold end-concentration) and incubated for one hour. Thereafter, one vial of Baby Rabbit complement (Cedarlane, Cat. No. CL3441) was reconstituted with 1 mL of Aqua bidest. The complement solution was diluted with medium and 25 μL added to the wells. After four hours the plates were centrifuged at 200 g and 100 μL/well were transferred to another 96-well flat bottom plate. Thereafter 100 μL of LDH reaction mix (Cytotoxicity Detection Kit, Roche Diagnostic GmbH, Mannheim, Germany) was added. After an incubation time of 20 min. at 37 °C optical density (OD) was measured at 492/690 nm on a Tecan Sunrise reader.

| sample | signal [OD] | |
|---|---|---|
| | BRC 1/40 | BRC 1/30 |
| 9000 ng/ml IL15-Fc-fusion with BRC | 11.3 | 12.3 |
| 3000 ng/ml IL15-Fc-fusion with BRC | 12.3 | 17.0 |
| 1000 ng/ml IL15-Fc-fusion with BRC | 10.2 | 13.6 |
| 333.3 ng/ml IL15-Fc-fusion with BRC | 7.8 | 12.2 |
| 111.1 ng/ml IL15-Fc-fusion with BRC | 8.3 | 13.0 |
| 37.04 ng/ml IL15-Fc-fusion with BRC | 14.9 | 19.7 |
| 12.35 ng/ml IL15-Fc-fusion with BRC | 43.2 | 53.0 |
| 4.12 ng/ml IL15-Fc-fusion with BRC | 41.5 | 63.8 |
| 0 ng/ml IL15-Fc-fusion with BRC | 42.4 | 48.4 |

It can be seen that BRC has a low background toxicity and shows dose dependent complement toxicity.

## Example 2

### C1q binding of anti-HER2 antibodies on BT-474 cells

About $3 \times 10^5$ BT-474 cells were incubated with 10 μg/mL of indicated antibody on ice in RPMI 1640 supplemented with 10 % FCS. After 30 min. incubation on ice 10 μg/mL C1q (Sigma Aldrich, Cat. No. C1740) was added. The incubation was continued thereafter for an additionally 20 min. on ice. After washing the cells were resuspended in 200 μL medium and counterstained with a PE-labeled antiC1q antibody (Cedarlane, Cat. No. CL7611PE-SP). After an incubation time of 30 min. on ice cells were washed twice and analyzed on a FACS Canto II.

| antibody/antibodies | PE-signal (geomean) |
|---|---|
| trastuzumab | 282 |
| pertuzumab | 344 |
| combination of trastuzumab and pertuzumab | 2157 |
| bispecific anti-HER2 antibody, common light chain | 1439 |
| bispecific anti-HER2 antibody, common light chain, glycoengineered | 1036 |
| bispecific anti-HER2 antibody, CrossMab format | 489 |

This C1q assay illustrates the binding of recombinant complement factor C1q to different antibodies on BT-474 cells.

**Example 3**

**Proliferation inhibition of anti-HER2 antibodies on BT-474 cells**

Ten thousand ($1 \times 10^4$) BT-474 cells/well were cultured in RPMI 1640 medium supplemented with 10 % FCS in a 96-well flat bottom plate. After 24 hours growth medium was removed and titrated amounts of indicated antibodies were added (premixed in culture medium; 200 nM, 66.7 nM, 22.2 nM, 7.4 nM, 2.5 nM, 0.8 nM, 0.3 nM, 0.1 nM) to a final volume of 100 $\mu$L. To determine the number of viable cells in culture, a CellTiterGlo Luminescent Cell Viability Assay according to the manufacturer's instructions was performed (quantifying ATP levels as an indicator of metabolically active cells). Thus, after six days of culture 100 $\mu$L CellTiterGlo Reaction Mix (Promega, Cat. No. G7571) was added to the cells and incubated for 2 min. with shaking. Thereafter 75 $\mu$L of the lysate was transferred to a separate 96-well flat bottom plate (Costar, Cat. No. 3917). After an additional mixing luminescence was assed according to the manufacturer's instructions using a Tecan Infinite Reader and the respective $IC_{50}$ value was calculated.

| antibody/antibodies | $IC_{50}$ [nM] |
|---|---|
| combination of trastuzumab and pertuzumab | 6.20 |
| bispecific anti-HER2 antibody, common light chain | 3.31 |
| bispecific anti-HER2 antibody, common light chain, glycoengineered | 3.93 |
| bispecific anti-HER2 antibody, CrossMab format | 4.75 |

In the proliferation assay it was shown that the antibodies inhibited proliferation of BT-474.

**Example 4**

**CDC activation by anti-HER2 antibodies on BT-474 cells, SK-Br3 cells and SK-OV-3 cells**

Ten thousand cells/well (BT-474, SK-Br3 or SK-OV-3 cells) were seeded in a 96-well plate and incubated for 20 hours at 37 °C/5 % $CO_2$. Thereafter the medium was removed, the cells were washed once with 100 $\mu$L AIM-V medium (Gibco, Cat. No. 0870112 DK). Fifty microliter AIM-V medium were placed in each well. Thereafter 50 $\mu$L antibody solution (in 3-fold end-concentration) were added and incubated for 30 min. at 37 °C/5 % $CO_2$. Fifty microliter of Baby Rabbit complement (Cedarlane, Cat. No. CL3441, batch no. 6312) 1:10 diluted in AIM-V medium was added and the incubation was continued for 2 hours. Thereafter, 50 $\mu$L of the supernatant was transferred and mixed with 50 $\mu$L LDH Reaction Mix (Roche Diagnostics GmbH, Mannheim, Germany). After a further incubation of 15 min. at 37 °C extinction (Ex.) was determined at 490/620 nm on a Tecan Sunrise Reader. The specific antibody dependent toxicity (mean +/- SD of n=4) was calculated as follows: % antibody dependent toxicity = (Ex. sample - Ex. spontaneous lysis / Ex. maximal lysis- spontaneous lysis) x 100. The results are shown in Figure 1.

BT474, SkBr3 and SK-OV-3 cells were incubated with trastuzumab, pertuzumab, or a combination thereof (total antibody concentration 10 $\mu$g/mL or 1 $\mu$g/mL), followed by a two hour incubation with Baby Rabbit complement. Human IgG1 with kappa light chain was used as isotype control. Readout of cell lysis (LDH release) was performed on a Tecan sunrise reader using the LDH Cytotoxicity kit (Roche Diagnostics GmbH, Mannheim, Germany, Cat. No. 11644793001). Specific lysis is given as the signal in relation to 3 % Triton-X treated cells (maximum lysis). Experiment was performed in quintuplicates.

| antibody/antibodies | dosage | specific lysis [%] | | |
|---|---|---|---|---|
| | | BT-474 cells | SkBr3 cells | SK-OV-3 cells |
| trastuzumab | 10 $\mu$g/mL | 12.8 ± 0.9 | -1.1 ± 0.7 | 0.5 ± 1.8 |
| pertuzumab | 10 $\mu$g/mL | 7.3 ± 0.6 | -1.4 ± 0.7 | -0.5 ± 1.1 |
| combination of trastuzumab and pertuzumab | 5 $\mu$g/mL + 5 $\mu$g/mL | 179.6 ± 1.3 | 157.2 ± 8.7 | 34.6 ± 9.9 |
| human IgG1, kappa | 10 $\mu$g/mL | 0.9 ± 0.8 | 5.2 ± 1.5 | -0.7 ± 1.0 |
| trastuzumab | 1 $\mu$g/mL | -8.1 ± 0.6 | -7.7 ± 3.1 | 1.3 ± 0.9 |

(continued)

| antibody/antibodies | dosage | specific lysis [%] | | |
|---|---|---|---|---|
| | | BT-474 cells | SkBr3 cells | SK-OV-3 cells |
| pertuzumab | 1 $\mu$g/mL | -5.1 $\pm$ 0.6 | -2.4 $\pm$ 0.2 | 1.5 $\pm$ 3.2 |
| combination of trastuzumab and pertuzumab | 0.5 $\mu$g/mL + 0.5 $\mu$g/mL | 109.3 $\pm$ 5.4 | 64.3 $\pm$ 19.8 | 20.9 $\pm$ 14.4 |
| human IgG1, kappa | 1 $\mu$g/mL | 10.3 $\pm$ 0.6 | 3.6 $\pm$ 1.0 | 1.5 $\pm$ 1.4 |

This CDC assay shows the release of LDH as a marker for dying/dead cells upon treatment with different antibodies (formats, combination) in the presence of Baby Rabbit complement.

**Example 5**

**Determination of antibody ratio for CDC**

Ten thousand SK-OV-3 cells per well were seeded into a 96-well flat bottom plate (Thermo Scientific, Nunclon Delta Surface) in 100 $\mu$L per well in AIM-V medium (Gibco, Cat. No. 0870112-DK) and were incubated for 20 hours at 37 °C and 5 % CO$_2$. After the incubation period, 50 $\mu$L of the antibody-stock solutions containing trastuzumab and pertuzumab at a final concentration of 0.1, 0.5, 1, 5, or 10 $\mu$g/mL were added. Human IgG1, kappa light chain (Sigma, Cat. No. I5154-1MG) was used as control. Triton-X (Roche Diagnostics GmbH, Mannheim, Germany, Cat. No. 11332481001) at a final concentration of 1 % was added for determination of maximum lysis. After incubation for 30 min. at 37 °C 50 $\mu$L Baby Rabbit Complement-stock solution (Cedarlane, Cat. No. CL3441) was added with a final dilution of 1/30. Thereafter the plates were incubated for 2 hours at 37 °C (final volume/well = 150 $\mu$L). The amount of cell lysis was determined via the LDH activity using the Cytotoxicity Detection Kit (Roche Diagnostics GmbH, Mannheim, Germany, Cat. No. 11644793001). The absorbance was determined at 490 nm and 620 nm with a Tecan Sunrise reader.
As positive control the following samples were used:

| | |
|---|---|
| medium control: | SK-OV-3 cells with AIM-V medium |
| spontaneous lysis: | SK-OV-3 cells with active BRC |
| maximal lysis: | SK-OV-3 cells with 1 % Triton-X |
| isotype control: | SK-OV-3 cells with 10 $\mu$g/mL human IgG, kappa and BRC |
| negative control: | SK-OV-3 cells with 10 $\mu$g/mL antibody/composition and heat inactivated BRC |
| assay control: | SK-OV-3 cells with 10 $\mu$g/mL trastuzumab and pertuzumab and active BRC. |

An optimal cell killing was observed at trastuzumab/pertuzumab ratios of 0.5:1 to 1:1 as well as at pertuzumab/trastuzumab ratio of 0.5:1 to 1:1. Overall, the assay seemed to be very robust towards the change of the antibody ratio since even a 1:10 ratio did not influence the CDC dramatically.

**Example 6**

**CDC-mediated killing of BT-474 cells by anti-HER2 antibodies**

Ten thousand BT-474 cells/well were seeded on 96-well E-Plates (ACEA Biosciences Inc.) and grown overnight in an Xcelligence device in AIM-V medium. Growth medium was removed and cells were washed once with serum-free AIM-V medium (Gibco). Fifty microliter per well AIM-V medium and 50 $\mu$L antibody in AIM-V (3-fold end concentration) were added and incubated for 20 min. Thereafter 50 $\mu$L Baby Rabbit complement (Cedarlane) was added and Cell Index (CI; as representative for the viability of the cells) was measured every 5 minutes. Specific CDC was calculated according following formula, whereas CI is the normalized cell index:

$$\% CDC = \frac{CI \text{ Complement control} - CI \text{ sample}}{CI \text{ Complement control}} \times 100$$

At two representative time points (1 hour and 2 hours after starting the reaction, specific lysis (i.e. CDC-induced cell death) was calculated and shown in Figure 2 and the following Table (mean+/SEM of n=4).

| antibody/antibodies | specific lysis [% cell index ACEA] | |
|---|---|---|
| | 1 hour | 2 hours |
| trastuzumab | -3.5 ± 0.6 | -6.5 ± 0.8 |
| pertuzumab | -5.3 ± 1.0 | -8.3 ± 2.1 |
| combination of trastuzumab and pertuzumab | 20.9 ± 6.7 | 26.3 ± 7.0 |
| bispecific anti-HER2 antibody, common light chain | 31.8 ± 3.4 | 38.9 ± 3.7 |
| bispecific anti-HER2 antibody, common light chain, glycoengineered | 28.8 ± 2.6 | 35.8 ± 2.6 |
| bispecific anti-HER2 antibody, CrossMab format | 12.9 ± 1.4 | 22.7 ± 1.6 |

This CDC assay illustrates a change in the cell index as a marker for dying/dead cells upon treatment with different antibodies (formats, combination) in the presence of Baby Rabbit complement.

## Example 7 (comparative example)

### Attempt to establish a CDC assay based on complement of human origin

SkBr3 cells were sensitized with trastuzumab, pertuzumab, or combination of trastuzumab and pertuzumab (10 μg/mL total antibody concentration) followed by a two hour incubation with Baby Rabbit complement (BCR, as described in Example 4) or with normal human serum (NHS) of three healthy donors (1:50 dilution, NHS 1, NHS 2, NHS 3). Human IgGlwith kappa light was used as isotype control.

Readout of cell lysis (LDH release) was performed on a Tecan sunrise reader using the LDH Cytotoxicity kit (Roche Diagnostics GmbH, Mannheim, Germany, Cat. No. 11644793001). Mean Lysis (in %) is the signal in relation to 3 % Triton-X treated cells (maximum lysis). Experiment was performed in triplicates.

| antibody/antibodies | specific lysis [%] | | | |
|---|---|---|---|---|
| | BRC | NHS 1 | NHS 2 | NHS 3 |
| trastuzumab | 12.5 ± 0.3 | 0.6 ± 0.6 | -2.9 ± 0.5 | -2.3 ± 0.6 |
| pertuzumab | 16.5 ± 0.9 | -1.9 ± 1.2 | -3.8 ± 0.4 | -4.0 ± 0.7 |
| combination of trastuzumab and pertuzumab | 46.9 ± 2.0 | 3.6 ± 1.5 | -0.6 ± 0.2 | -0.9 ± 1.5 |
| human IgG1, kappa | 4.9 ± 1.2 | -3.5 ± 0.8 | -6.8 ± 1.7 | -5.2 ± 0.7 |

## Example 8 (comparative example)

### siRNA mediated knockdown of CD55, CD59 and CD46

### Generation of cell lines

For the CD46, CD55 and CD59 knockdown, SK-OV-3 cells were treated with corresponding siRNA (Biospring; CD46 Cat. No. 203525-A, CD55 Cat. No. 203526-A, CD59 Cat. No. 203527-A), one control siRNA (Biospring, Cat. No. 203524-A) and the transfection reagent LipofectAmine (Invitrogen, Cat. No. 13778-100). The quantities used were according to the manufacturer's protocol. After three days of cultivation the amount of CD46, CD55 and CD59 on the cell surface was determined by FACS-analysis using a cell suspension with 1-2 x10$^5$ cells in 50 μL and master mix of FACS-antibodies. The antibody-master mix contained 1 μL each of anti-CD-55-APC antibody (BD Pharmingen, Cat. No. 555696) and anti-CD59-PE antibody (BD Pharmingen, Cat. No. 555764) and 10 μL of anti-CD46-FITC antibody (BD Pharmingen, Cat. No. 555949), 10 % mouse serum (Southern Biotech, Cat. No. 0050-01) and FACS-Buffer (5 mL DPBS supplemented with 20 μL BSA). The FACS antibodies were titrated to determine the appropriate concentration to be employed. For isotype control, 20 μL IgG2a,k-FITC (BD Pharmingen, Cat. No. 556652), IgG2a,k-APC (BD Pharmingen, Cat. No. 552893), IgG2a,k-PE (BD Pharmingen, Cat. No. 551438) each with 10% mouse serum and FACS-Buffer were used. Cells were incubated with the above-mentioned FACS-antibodies for 30 minutes at 4 °C and 20 rpm, washed with 600 µL ice-cold DPBS buffer and resuspended in 200 µL Cytofix (BD Pharmingen, Cat. No. 554655). The FACS analysis was performed on a FACS Canto II.

| target | signal | wild-type SK-OV-3 cells | knockdown SK-OV-3 cells |
|--------|--------|-------------------------|-------------------------|
| CD46 | FITC | 683 | 662 |
| CD55 | APC | 1447 | 275 |
| CD59 | PE | 1192 | 649 |

A significant knockout was achieved for CD 55 (about 80 % knockdown). The expression of CD 59 was down-regulated by about 45 %. CD46 shows no change in the expression level.

CDC after knockdown

For CD46, CD55 and CD59 knockdown, SK-OV-3 cells were treated with the corresponding siRNAs (Biospring; CD46 Cat. No. 203525-A, CD55 Cat. No. 203526-A, CD59 Cat. No. 203527-A) and the transfection reagent LipofectAmine (Invitrogen, Cat. No. 13778-100). The quantities used were according to the manufacturer's protocol. After three days of cultivation the amount of CD46, CD55 and CD59 on the cell surface was determined by FACS-analysis (see above). At day four a CDC-assay was performed with wild-type (= non-siRNA treated) SK-OV-3, SK-OV-3-triple cells (transfected with all three siRNAs) and SK-OV-3-Contrl.siRNA (transfected with an unspecific control siRNA). For the CDC-Assay 10.000 cells per well were seeded into a 96-well flat bottom plate (Thermo Scientific, Nunclon Delta Surface) containing 100 µL per well in AIM-V medium (Gibco, Cat. No. 0870112-DK) and were incubated for 20 hours at 37 °C and 5 % $CO_2$. Thereafter trastuzumab, pertuzumab, human IgG1, kappa (Sigma, Cat. No. I5154) and bispecific anti-HER2 antibody (common light chain) were tested at a final concentration of 10 µg/mL. Triton-X (Roche Diagnostics GmbH, Mannheim, Germany, Cat. No. 11332481001) at a final concentration of 1 % was used for the determination of the maximal lysis. All samples were incubated for 30 min. at 37 °C. Subsequently, Baby Rabbit complement (BRC) (Cedarlane, Cat. No. CL3441) and Normal Human Serum (NHS) was added at a final dilution of 1/30 and the plates were incubated for 2 hours at 37 °C (final volume/well = 150 µL). The amount of cell lysis was determined via LDH activity using the Cytotoxicity Detection Kit (Roche Diagnostics GmbH, Mannheim, Germany, Cat. No. 11644793001). The absorbance was determined at 490 nm and 620 nm using a Tecan Sunrise reader.

As positive control the following samples were used:

medium control:　　　SK-OV-3 cells with AIM-V medium
spontaneous lysis:　　SK-OV-3 cells with active BRC
maximal lysis:　　　　SK-OV-3 cells with 1 % Triton-X
isotype control:　　　SK-OV-3 cells with 10 µg/mL human IgG, kappa and BRC
negative control:　　　SK-OV-3 cells with 10 µg/mL antibody/composition and heat inactivated BRC
assay control:　　　　SK-OV-3 cells with 10 µg/mL trastuzumab and pertuzumab and active BRC.

The results are shown in Figure 4.

In the presence of NHS as source of complement the knockdown of CD55 and CD59 is absolutely required to exert CDC. The tedious siRNA knockdown procedure can be overcome by the use of BRC. The assay showed no influence by the presence of mCRPs on the carcinoma cells. This is the prerequisite for using the assay as reported herein for high throughput screening of different antibody formats (besides the screening for different antibody combinations) or plain as a positive control for other CDC assays.

The positive control showed that the CDC assay was working. The comparison of the OD 490/620 nm and the specific cytotoxicity (%) of SK-OV-3, SK-OV-3-triple-KO and SK-OV-3-Contrl.siRNA showed that the control siRNA does not induce cytotoxicity.

**Example 9 (comparative example)**

**Attempt to establish a CDC assay by manipulation of membrane bound complement regulatory proteins (mCRPs)**

To elucidate whether restrictive factors by the target cells specifically against NHS (as opposed to BRC) influence the assay it was attempted to decrease the expression of mCRPs, a group of proteins inhibiting different stages of the CDC process.

anti-CD55 and anti-CD59 neutralizing antibodies

mCRP inhibition was performed using neutralizing antibodies directed against the targets CD55 and CD59 in single and combined incubation (total neutralizing antibody concentration 10 μg/mL) after treatment with trastuzumab and/or pertuzumab (see Zhao, W.P., et al., One. Rep. 21 (2009) 1405-1411).

The assay was performed as in Example 6 with the following adaptations: SK-OV-3 cells were incubated with neutralizing antibody (neu mAb) against CD55, CD59 or both (total neu mAb concentration 10 μg/mL), followed by incubation with trastuzumab and/or pertuzumab (or isotype control) and addition of serum (1:30 dilution). The observations are shown in Figure 3.

The experiment was repeated with pooled human serum with the same outcome.

In both experiments BRC resulted in about 50 % relative CDC whereas with human serum less than 15 % relative CDC could be observed.

siRNA-mediated knockdown of CD46, CD55 and CD59

Decrease of mCRP expression was performed by RNA interference technology (RNAi) by transfecting mCRP-mRNA specific small interfering RNA (siRNA) molecules into the cells which will lead to the degradation of the respective mRNA molecules in the cytoplasm. Target cells were subjected to FACS analysis for surface expression of mCRPs 48h after siRNA transfection to determine the efficiency of the gene knockdown (see e.g. Mamidi, S., et al., Mol. One.7 (2013) 580-594).

BT474, SkBr3 and SK-OV-3 cells were transfected with CD46 (SEQ ID NO: 12 and 13), CD55 (SEQ ID NO: 14 and 15), CD59 (SEQ ID NO: 16 and 17) or scrambled siRNAs (SEQ ID NO: 18 and 19) (usually 25 nM) using lipofection (Dharmafect 1) according to the manufacturer's protocol (Biospring).

Forty-eight hours post transfection $2 \times 10^5$ cells were stained with the labelled antibodies against CD46, CD55 and CD59 (5 μg/mL each; CD46-FITC Cat. No. 555949, CD55-APC Cat. No. 555696, CD59-PE Cat. No. 555764; BD Pharmingen) and the surface expression of the indicated receptors including controls were analyzed. The relative reduction in CD46 expression as determined by FACS is shown in the following Table.

| (n=2) | BT-474 cells | SkBr3 cells | SK-OV-3 cells |
|---|---|---|---|
| **RNA** | [rel. reduction MFI FACS] | | |
| untreated reference | 0 | 0 | 0 |
| mock transfected | 4.2 | -8.6 | -4.9 |
| scrambled | 10.5 | -1.7 | -8.6 |
| CD46-siRNA | 67.4 | 42.7 | 55.4 |

The relative reduction in CD46, CD55 and CD59 expression as determined by FACS of triple siRNA transfected cells is shown in the following Table.

| siRNA | BT-474 cells | SkBr3 cells | SK-OV-3 cells |
|---|---|---|---|
| | [rel. reduction MFI FACS] | | |
| CD46 | 64.6 | 1.4 | 55.1 |
| CD55 | 26.5 | 69.6 | 61.7 |
| CD59 | 72.5 | -45.4(*) | 88.0 |
| (*) SK-OV-3 cells are very sensitive for siRNA transfection. Mock transfection and scrambled RNA transfected resulted in an increase of CD46 expression by up to 27 % and of CD59 expression of up to 610 %. | | | |

LDH assay of SkBr3 cells was performed 96 hours post transfection as described in Example 4 using Baby Rabbit complement or pooled Normal Human Serum (in the presence or absence of mentioned siRNA or controls). In the following table is shown the means percentage of specific lysis of SkBr3 cells transfected with siRNA for CD46, CD55 and CD59 (triple transfected cells) with Baby Rabbit complement (+/- SD of n=3).

| antibody/antibodies | specific lysis [%] |
|---|---|
| trastuzumab | 0.2 ± 4.2 |
| pertuzumab | -5.3 ± 1.6 |
| combination of trastuzumab and pertuzumab | 52.0 ± 10.3 |
| human IgG1, kappa | -6.5 ± 2.1 |

In the following table is shown the means percentage of specific lysis of SkBr3 cells transfected with siRNA for CD46, CD55 and CD59 (triple transfected cells) with pooled Human serum (+/- SD of n=3).

| antibody/antibodies | specific lysis [%] |
|---|---|
| trastuzumab | 9.7 ± 1.7 |
| pertuzumab | -1.2 ± 2.0 |
| combination of trastuzumab and pertuzumab | 8.7 ± 1.4 |
| human IgG1, kappa | 5.0 ± 1.8 |

**Claims**

1.  A method for determining complement dependent cytotoxicity of a composition
    wherein the composition comprises

    i) a first binding site that specifically binds to a first epitope on a first antigen, which is conjugated to a first Fc-region polypeptide of human origin, and
    ii) a second binding site that specifically binds to a second epitope on the first antigen, which is conjugated to a second Fc-region polypeptide of human origin,

    wherein the method comprises the following steps:

    a) incubating a human carcinoma cell of epithelial origin expressing the first antigen with the composition,
    b) adding rabbit complement to the mixture of a), and
    c) determining cell lysis and thereby determining complement dependent cytotoxicity of the composition.

2.  The method according to claim 1, wherein the composition binds to a first epitope on the first antigen and a second epitope on the first antigen and the first epitope and the second epitope are different.

3.  The method according to claim 2, wherein the first epitope and the second epitope are non-overlapping epitopes.

4.  The method according to any one of claims 1 to 3, wherein cell lysis is determined between 0.5 and 3 hours after the addition of complement.

5.  The method according to any one of claims 1 to 4, wherein the method is a serum-free method.

6.  The method according to any one of claims 1 to 5, wherein the rabbit complement is Baby Rabbit complement.

7.  The method according to any one of claims 1 to 6, wherein the ratio of the first binding site to the second binding site is of from 0.5:1 to 1:0.5.

**Patentansprüche**

**1.** Verfahren zur Bestimmung Komplement-abhängiger Zytotoxizität einer Zusammensetzung, wobei die Zusammensetzung Folgendes umfasst

> i) eine erste Bindungsstelle, die spezifisch an ein erstes Epitop an einem ersten Antigen bindet und die an ein erstes Fc-Region-Polypeptid humanen Ursprungs konjugiert ist, und
> ii) eine zweite Bindungsstelle, die spezifisch an ein zweites Epitop an dem ersten Antigen bindet und die an ein zweites Fc-Region-Polypeptid humanen Ursprungs konjugiert ist,

wobei das Verfahren die folgenden Schritte umfasst:

> a) Inkubieren einer humanen Karzinomzelle epithelialen Ursprungs, die das erste Antigen exprimiert, mit der Zusammensetzung,
> b) Zugeben von Kaninchen-Komplements zu dem Gemisch aus a), und
> c) Bestimmung der Zelllyse und dabei Bestimmung der Komplement-abhängigen Zytotoxizität der Zusammensetzung.

**2.** Verfahren nach Anspruch 1, wobei die Zusammensetzung an ein erstes Epitop an dem ersten Antigen und ein zweites Epitop an dem ersten Antigen bindet und das erste Epitop und das zweite Epitop verschieden sind.

**3.** Verfahren nach Anspruch 2, wobei das erste Epitop und das zweite Epitop nichtüberlappende Epitope sind.

**4.** Verfahren nach einem der Ansprüche 1 bis 3, wobei die Zelllyse zwischen 0,5 und 3 Stunden nach der Zugabe des Komplements bestimmt wird.

**5.** Verfahren nach einem der Ansprüche 1 bis 4, wobei das Verfahren ein serumfreies Verfahren ist.

**6.** Verfahren nach einem der Ansprüche 1 bis 5, wobei das Kaninchen-Komplement Baby-Kaninchen-Komplement ist.

**7.** Verfahren nach einem der Ansprüche 1 bis 6, wobei das Verhältnis der ersten Bindungsstelle zur zweiten Bindungsstelle 0,5:1 bis 1:0,5 ist.


**Revendications**

**1.** Procédé de détermination de la cytotoxicité dépendante du complément d'une composition dans lequel la composition comprend

> i) un premier site de liaison qui se lie spécifiquement à un premier épitope sur un premier antigène, qui est conjugué à un premier polypeptide de région Fc d'origine humaine, et
> ii) un second site de liaison qui se lie spécifiquement à un second épitope sur le premier antigène, qui est conjugué à un second polypeptide de région Fc d'origine humaine,

dans lequel le procédé comprend les étapes suivantes :

> a) incuber une cellule de carcinome humain d'origine épithéliale exprimant le premier antigène avec la composition,
> b) ajouter du complément de lapin au mélange de a), et
> c) déterminer la lyse cellulaire et déterminer ainsi la cytotoxicité dépendante du complément de la composition.

**2.** Procédé selon la revendication 1, dans lequel la composition se lie à un premier épitope sur le premier antigène et à un second épitope sur le premier antigène et le premier épitope et le second épitope sont différents.

**3.** Procédé selon la revendication 2, dans lequel le premier épitope et le second épitope sont des épitopes non chevauchants.

4. Procédé selon l'une quelconque des revendications 1 à 3, dans lequel la lyse cellulaire est déterminée entre 0,5 et 3 heures après l'ajout du complément.

5. Procédé selon l'une quelconque des revendications 1 à 4, dans lequel le procédé est un procédé exempt de sérum.

6. Procédé selon l'une quelconque des revendications 1 à 5, dans lequel le complément de lapin est un complément de bébé lapin.

7. Procédé selon l'une quelconque des revendications 1 à 6, dans laquelle le rapport du premier site de liaison au second site de liaison est de 0,5:1 à 1:0,5.

Figure 1

Figure 2

**Figure 3**

# Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5851528 A
- US 8034902 B
- US 20120226020 A
- US 20130302355 A
- US 20100255011 A
- WO 2008007648 A
- WO 2010120541 A
- WO 0042072 A
- US 20080089892 A
- WO 2006105062 A
- US 5821337 A
- US 7862817 B
- US 7527791 B
- US 6982321 B
- US 7087409 B
- WO 9308829 A
- US 5731168 A
- WO 2009089004 A
- US 4676980 A
- US 20060025576 A
- US 20080069820 A
- WO 2009080251 A
- WO 2009080252 A

- WO 2009080253 A
- WO 2009080254 A
- WO 2010112193 A
- WO 2010115589 A
- WO 2010136172 A
- WO 2010145792 A
- WO 2010145793 A
- US 4816567 A
- US 5648237 A
- US 5789199 A
- US 5840523 A
- US 5959177 A
- US 6040498 A
- US 6420548 B
- US 7125978 B
- US 6417429 B
- US 20050260186 A
- US 20060104968 A
- US 6267958 B
- US 6171586 B
- WO 2006044908 A
- EP 14198749

**Non-patent literature cited in the description**

- **SLEDGE et al.** *J. Biol. Chem.,* 1973, vol. 248, 2818-2813
- **HUGHES-JONES et al.** *Mol. Immunol.,* 1979, vol. 16, 697-701
- **BURTON et al.** *Mol. Immunol.,* 1990, vol. 22, 161-206
- **PERKINS et al.** *Biochem. J.,* 1985, vol. 228, 13-26
- **POON et al.** *J. Mol. Biol.,* 1983, vol. 168, 563-577
- **REID et al.** *Biochem. Soc. Trans.,* 1983, vol. 11, 1-12
- **WEISS et al.** *J. Mol. Biol.,* 1986, vol. 189, 573-581
- *Nature Sci. Rep.,* 2011, vol. 1, 1-11
- **HERVE, P. et al.** *Transplant.,* 1985, vol. 39, 138-143
- **VESSELLA, R.L. et al.** *Canc. Res.,* 1985, vol. 45, 6131-6139
- **HELLSTROEM, I. et al.** *Int. J. Canc.,* 1983, vol. 31, 553-555

- **NUUTILA, J. et al.** *Hum. Immunol.,* 2013, vol. 74, 522-530
- **FISHELSON, Z. et al.** *Mol. Immunol.,* 2003, vol. 40, 109-123
- **DECHANT et al.** *Cancer Research,* 2008
- **MAMIDI, S. et al.** *Mol. Onc.,* 2013, vol. 7, 580-594
- *Clin. Vac. Immunol.,* 2008, vol. 15, 88-94
- *Brit. J. Hematol.,* 2013, vol. 163, 182-193
- *J. Immunol.,* 1981, vol. 127, 157-160
- *Clin. Cancer Res.,* 2007, vol. 13, 4556-4564
- *FEMS Immunol. Med. Microbiol.,* 2001, vol. 31, 203-209
- *Blood,* 2006, vol. 108, 713a-714a
- *Clin. Vacc. Immunol.,* 2008, vol. 15, 88-94
- *Brit. J. Haematol.,* 2013, vol. 163, 182-193
- *Blood,* 2006, vol. 108, 713A-714A
- **KABAT, E.A. et al.** Sequences of Proteins of Immunological Interest. Public Health Service, National Institutes of Health, 1991

- **CHOTHIA, C. ; LESK, A.M.** *J. Mol. Biol.,* 1987, vol. 196, 901-917
- **MACCALLUM et al.** *J. Mol. Biol.,* 1996, vol. 262, 732-745
- **FLATMAN, S. et al.** *J. Chromatogr. B,* 2007, vol. 848, 79-87
- **KABAT et al.** Sequences of Proteins of Immunological Interest. Public Health Service, National Institutes of Health, 1991
- **ALMAGRO, J.C. ; FRANSSON, J.** *Front. Biosci.,* 2008, vol. 13, 1619-1633
- **RIECHMANN, I. et al.** *Nature,* 1988, vol. 332, 323-329
- **QUEEN, C. et al.** *Proc. Natl. Acad. Sci. USA,* 1989, vol. 86, 10029-10033
- **KASHMIRI, S.V. et al.** *Methods,* 2005, vol. 36, 25-34
- **PADLAN, E.A.** *Mol. Immunol.,* 1991, vol. 28, 489-498
- **DALL'ACQUA, W.F. et al.** *Methods,* 2005, vol. 36, 43-60
- **OSBOURN, J. et al.** *Methods,* 2005, vol. 36, 61-68
- **KLIMKA, A. et al.** *Br. J. Cancer,* 2000, vol. 83, 252-260
- **SIMS, M.J. et al.** *J. Immunol.,* 1993, vol. 151, 2296-2308
- **CARTER, P. et al.** *Proc. Natl. Acad. Sci. USA,* 1992, vol. 89, 4285-4289
- **PRESTA, L.G. et al.** *J. Immunol.,* 1993, vol. 151, 2623-2632
- **BACA, M. et al.** *J. Biol. Chem.,* 1997, vol. 272, 10678-10684
- **ROSOK, M.J. et al.** *J. Biol. Chem.,* vol. 271, 22611-22618
- **MILSTEIN, C. ; CUELLO, A.C.** *Nature,* 1983, vol. 305, 537-540
- **TRAUNECKER, A. et al.** *EMBO J.,* 1991, vol. 10, 3655-3659
- **BRENNAN, M. et al.** *Science,* 1985, vol. 229, 81-83
- **KOSTELNY, S.A. et al.** *J. Immunol.,* 1992, vol. 148, 1547-1553
- **HOLLIGER, P. et al.** *Proc. Natl. Acad. Sci. USA,* 1993, vol. 90, 6444-6448
- **GRUBER, M. et al.** *J. Immunol.,* 1994, vol. 152, 5368-5374
- **TUTT, A. et al.** *J. Immunol.,* 1991, vol. 147, 60-69
- **CHARLTON, K.A.** Methods in Molecular Biology. Humana Press, 2003, vol. 248, 245-254
- **GERNGROSS, T.U.** *Nat. Biotech.,* 2004, vol. 22, 1409-1414
- **LI, H. et al.** *Nat. Biotech.,* 2006, vol. 24, 210-215
- **GRAHAM, F.L. et al.** *J. Gen Virol.,* 1977, vol. 36, 59-74
- **MATHER, J.P.** *Biol. Reprod.,* 1980, vol. 23, 243-252
- **MATHER, J.P. et al.** *Annals N.Y. Acad. Sci.,* 1982, vol. 383, 44-68
- **URLAUB, G. et al.** *Proc. Natl. Acad. Sci. USA,* 1980, vol. 77, 4216-4220
- **YAZAKI, P. ; WU, A.M.** Methods in Molecular Biology. Humana Press, 2004, vol. 248, 255-268
- Remington's Pharmaceutical Sciences. 1980
- **SAMBROOK, J. et al.** Molecular cloning: A laboratory manual. Cold Spring Harbor Laboratory Press, 1989
- **ZHAO, W.P. et al.** *One. Rep.,* 2009, vol. 21, 1405-1411
- **MAMIDI, S. et al.** *Mol. One.,* 2013, vol. 7, 580-594